# EUROPEAN PATENT SPECIFICATION

(11) **EP 1 128 730 B1**
(45) Date of publication and mention of the grant of the patent: **02.05.2003**
(21) Application number: 99953735.0
(22) Date of filing: 09.11.1999
(51) Int. Cl.: A01N 63/00, A01N 37/00, A01N 59/00, C11D 3/386, G02C 13/00, A61K 7/00

(54) **ANTIMICROBIAL COMPOSITION COMPRISING AN OXIDOREDUCTASE AND AN ENHANCING AGENT OF THE N-HYDROXYANILIDE-TYPE**
ANTIMIKORBIELLE ZUSAMMENSETZUNG, DIE EINE OXIDOREDUKTASE UND EIN VERSTÄRKUNGSMITTEL VOM N-HYDROXYANILID-TYP ENTHÄLT
COMPOSITION ANTIMICROBIENNE A BASE D'OXYDOREDUCTASE ET D'AGENT FACILITANT DU TYPE N-HYDROXYANILIDE

(30) Priority: 09.11.1998 DK 144198
(43) Date of publication of application: 05.09.2001
(73) Proprietor: Novozymes A/S, 2880 Bagsvaerd (DK)
(72) Inventor: JOHANSEN, Charlotte, DK-2840 Holte (DK); DEUSSEN, Heinz-Josef, DK-2860 S borg (DK)
(74) Representative: Stahr, Pia
(86) International application number: DK9900609
(87) International publication number: WO00027204

(56) References cited:
- EP-A1- 0 825 294
- WO-A1-96/38548
- WO-A1-97/42825
- WO-A1-97/48786
- WO-A1-98/51772
- WO-A1-99/08531
- WO-A2-94/29425

## Description

### FIELD OF THE INVENTION

The present invention relates to an enzymatic composition capable of killing or inhibiting microbial cells or micro-organisms present, e.g., in laundry, on hard surfaces, in water systems, on skin, on teeth or on mucous membranes. The present invention also relates to the use of said enzymatic composition for preserving food products, cosmetics, paints, coatings, etc.

### BACKGROUND OF THE INVENTION

Various enzymatic antimicrobial compositions are known in the art. For instance, WO 94/04127 discloses stabilized dentifrice compositions which are capable of producing antimicrobially effective concentrations of hypothiocyanite ions. The compositions contain an oxidoreductase capable of producing hydrogen peroxide and a peroxidase enzyme capable of oxidizing thiocyanate ions normally present in saliva to antimicrobial hypothiocyanite ions. Suitable peroxidases include lactoperoxidase, myeloperoxidase, salivary peroxidase and chloroperoxidase.

In EP-A-0 500 387 enzymatic antimicrobial compositions are disclosed comprising a haloperoxidase, e.g., myelo-peroxidase, eosinophil oxidase, lactoperoxidase and chloroperoxidase, which selectively binds to and inhibits the growth of target micro-organisms in the presence of peroxide and halide.

WO 95/27046 discloses an antimicrobial composition comprising a Vanadium chloroperoxidase, halide ions, and hydrogen peroxide or a hydrogen peroxide-generating agent.

WO 96/38548 discloses an antimicrobial composition comprising a haloperoxidase, a halide ion, a peroxide generating agent and an amino acid.

WO 97/42825 discloses an antimicrobial composition comprising a peroxidase, a hydrogen peroxide source and an enhancing agent of the phenothiazine-type or of the acetosyringate-type.

WO 94/29425 discloses a multicomponent bleaching system for use in detergent substances, consisting of oxidation catalysts, suitable oxidizing agents and aliphatic, cycloaliphatic, heterocyclic or aromatic compounds containing NO, NOH or NRHOH.

EP 825294 discloses a multicomponent system for modifying, degrading or bleaching lignin, lignin-containing materials or similar substances, comprising (a) at least one enzyme; (b) at least one suitable oxidizing agent; and (c) at least one N-aryl-N-hydroxyamide mediator.

WO 97/48786 discloses a multicomponent system for use with detergent substances, containing (a) optionally at least one oxidation catalyst; (b) at least one suitable oxidant; (c) at least one mediator selected from the group of hydroxylamines, hydroxylamine derivatives, hydroxamic acids, hydroxamic acid derivatives, of aliphatic, cycloaliphatic, heterocyclic or aromatic compounds which have at least one N-hydroxy function, oxime function, N-oxi-function, or N,N'-dioxi function; (d) at least one co-mediator selected from the group of aryl-substituted alcohols, carbonyl compounds, aliphatic ethers, phenol ethers and/or olefines(alkenes).

WO 98/51772 discloses an enzymatic bleaching system to be used with detergent substances containing a) at least one oxidation catalyst, b) at least one oxidizing agent, c) at least one mediator from the amide group, and/or from the imide group, and/or from the oxocarbon group.

WO 99/08531 discloses enzymatic antimicrobial composition comprising a haloperoxidase, a hydrogen peroxide source, a halide source, and an ammonium source, in particular an ammonium salt or an aminoalcohol.

The object of the present invention is to provide a composition for killing or inhibiting microbial cells.

### SUMMARY OF THE INVENTION

According to the invention it has been found that an antimicrobial composition comprising a phenol oxidizing enzyme system and an enhancing agent comprising a -CO-NOH- group is very effective for killing or inhibiting microbial cells.

Thus, based on these findings, the present invention provides as a first aspect:

A method for killing or inhibiting microbial cells comprising treating said microbial cells with a composition comprising a laccase or a laccase related enzyme together with oxygen and an enhancing agent of the following formula: 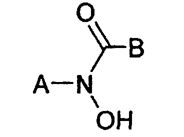 in which A and B independently of each other are: 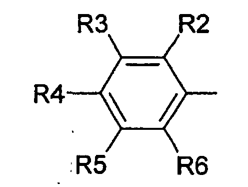 or B is H or C₁₋₁₆-alkyl, said alkyl may contain hydroxy, ether or ester groups, and R2, R3, R4, R5 and R6 independently of each other are H, OH, NH₂, COOH, SO₃H, C₁₋₁₂-alkyl, acyl, NO₂, CN, Cl, CF₃, NOH-CO-phenyl, CO-NOH-phenyl, C₁₋₆-CO-NOH-A, CO-NOH-A, COR12, phenyl-CO-NOH-A, OR7, NR8R9, COOR10, or NOH-CO-R11, wherein R7, R8, R9, R10, R11 and R12 are C₁₋₁₂-alkyl or acyl.

In further aspects, the present invention relates to methods for killing or inhibiting microbial cells, e.g. in laundry, in cosmetic products or on hard surfaces.

In a further aspect, the present invention relates to the composition of the method of the invention, wherein the composition in an aqueous solution has a pH in the range of pH 5 to 10.5.

In still further aspects, the present invention relates to use of an enzymatic antimicrobial composition for cleaning of contact lenses, for cleaning of water systems, for preserving of paint, and in a cleaning-in-place system.

### BRIEF DESCRIPTION OF DRAWINGS

The present invention is further illustrated by reference to the accompanying drawings, in which:
Fig. 1 shows the antimicrobial activity of *C. cinereus* peroxidase against *P. fluorescens.* (Peroxidase: 3 POXU/ml, Enhancing agent: 200 µM N-hydroxyacetanilide; see Example 1). ■ = pH 8; □ = pH 6; ---- = total kill.

Fig. 2 shows the dosis-response curve for N-hydroxyacetanilide in combination with *Polyporus laccase* (rPpL) at pH 6, 20 min and 40°C (see Example 2). ―□― = *Enterococcus faecalis*; --○-- = Pseudomonas aeruginosa; ···Δ··· = *Enterobacter aerogenes.*

Fig. 3 shows the dosis-response curve for N-hydroxyacetanilide in combination with *Coprinus* laccase (rCcL) at pH 6, 20 min and 40°C (see Example 2). ―□― = *Enterococcus faecalis;* --○-- = *Pseudomonas aeruginosa;* ···Δ··· = *Enterobacter aerogenes.*

### DETAILED DESCRIPTION OF THE INVENTION

In the context of the present invention the term "antimicrobial" is intended to mean that there is a bactericidal and/or a bacteriostatic and/or fungicidal and/or fungistatic effect and/or a virucidal effect and/or a sporicidal effect, wherein

The term "bactericidal" is to be understood as capable of killing bacterial cells.

Bactericidal activity is measured as a logarithmic reduction (log reduction) in the number of living cells or Colony Forming Units pr. ml (CFU/ml), e.g. 1 log reduction corresponds to a reduction in the number of living cells of *Escherichia coli* DSM1576 or *Enterococcus faecalis* DSM2570 from Y x 10^{X} CFU/M (CPU: Colony Forming Units; M: ml or g) to Y x 10^{X-1} CFU/M, where X can be 1, 2, 3, 4, 5, 6, 7, 8, 9, 10 or 11, and Y can be any number from 0 to 10. The number of living cells are to be determined as the number of *E. coli* or *E. faecalis,* respectively, which can grow on Tryptone Soya Agar (#CM129, Oxoid, England) plates at 30°C.

The term "bacteriostatic" is to be understood as capable of inhibiting bacterial growth, i.e. inhibiting growing bacterial cells.

The term "fungicidal" is to be understood as capable of killing fungal cells.

The term "fungistatic" is to be understood as capable of inhibiting fungal growth, i.e. inhibiting growing fungal cells.

The term "virucidal" is to be understood as capable of inactivating virus.

The term "sporicidal" is to be understood as capable of inactivating spores.

The term "microbial cells" denotes bacterial or fungal cells, and the term "micro-organism" denotes a fungus (including yeasts) or a bacterium.

In the context of the present invention the term "inhibiting growth of microbial cells" is intended to mean that the cells are in the non-growing state, i.e., that they are not able to propagate.

The term "hard surface" as used herein relates to any surface which is essentially non-permeable for micro-organisms. Examples of hard surfaces are surfaces made from metal, e.g., stainless steel, plastics, rubber, board, glass, wood, paper, textile, concrete, rock, marble, gypsum and ceramic materials which optionally may be coated, e.g., with paint, enamel and the like. The hard surface can also be a process equipment, e.g., a cooling tower, an osmotic membrane, a water treatment plant, a dairy, a food processing plant, a chemical or pharmaceutical process plant. Accordingly, the composition according to the present invention is useful in a conventional cleaning-in-place (C-I-P) system.

### Enhancing agents

The present invention relates to enhancing agents comprising a -CO-NOH- group of the following formula: 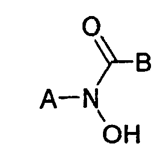 in which A and B independently of each other are: 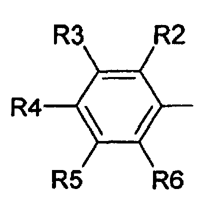 or B is H or C₁₋₁₆-alkyl, said alkyl may contain hydroxy, ester or ether groups (e.g. wherein the ether oxygen is directly attached to A-N(OH)C=O-, thus including N-hydroxy carbamic acid ester derivatives), and R2, R3, R4, R5 and R6 independently of each other are H, OH, NH₂, COOH, SO₃H, C₁₋₁₂-alkyl, acyl, NO₂, CN, Cl, Br, F, CF₃, NOH-CO-phenyl, CO-NOH-phenyl, C₁₋₆-CO-NOH-A, CO-NOH-A, COR12, phenyl-CO-NOH-A, OR7, NR8R9, COOR10, or NOH-CO-R11, wherein R7, R8, R9, R10, R11 and R12 are C₁₋₁₂-alkyl or acyl.

R2, R3, R4, R5 and R6 of A are preferably H, OH, NH₂, COOH, SO₃H, C₁₋₃-alkyl, acyl, NO₂, CN, Cl, Br, F, CF₃, NOH-CO-phenyl, CO-NOH-phenyl, COR12, OR7, NR8R9, COOR10, or NOH-CO-R11, wherein R7, R8 and R9 are C₁₋₃-alkyl or acyl, and R10, R11 and R12 are C₁₋₃-alkyl; more preferably R2, R3, R4, R5 and R6 of A are H, OH, NH₂, COOH, SO₃H, CH₃, acyl, NO₂, CN, Cl, Br, F, CF₃, CO-NOH-phenyl, COCH₃, OR7, NR8R9, or COOCH₃, wherein R7, R8 and R9 are CH₃ or COCH₃; even more preferably R2, R3, R4, R5 and R6 of A are H, OH, COOH, SO₃H, CH₃, acyl, NO₂, CN, Cl, Br, F, CO-NOH-phenyl, OCH₃, COCH₃, or COOCH₃; and in particular R2, R3, R4, R5 and R6 of A are H, OH, COOH, SO₃H, CH₃, NO₂, CN, Cl, Br, CO-NOH-phenyl, or OCH₃.

R2, R3, R4, R5 and R6 of B are preferably H, OH, NH₂, COOH, SO₃H, C₁₋₃-alkyl, acyl, NO₂, CN, Cl, Br, F, CF₃, NOH-CO-phenyl, CO-NOH-phenyl, COR12, OR7, NR8R9, COOR10, or NOH-CO-R11, wherein R7, R8 and R9 are C₁₋₃-alkyl or acyl, and R10, R11 and R12 are C₁₋₃-alkyl; more preferably R2, R3, R4, R5 and R6 of B are H, OH, NH₂, COOH, SO₃H, CH₃, acyl, NO₂, CN, Cl, Br, F, CF₃, CO-NOH-phenyl, COCH₃, OR7, NR8R9, or COOCH₃, wherein R7, R8 and R9 are CH₃ or COCH₃; even more preferably R2, R3, R4, R5 and R6 of B are H, OH, COOH, SO₃H, CH₃, acyl, NO₂, CN, Cl, Br, F, CO-NOH-phenyl, OCH₃, COCH₃, or COOCH₃; and in particular R2, R3, R4, R5 and R6 of B are H, OH, COOH, SO₃H, CH₃, NO_{2,} CN, Cl, Br, CO-NOH-phenyl, or OCH₃.

B is preferably H or C₁₋₃-alkyl, said alkyl may contain hydroxy, ester or ether groups; preferably said alkyl may contain ester or ether groups; more preferably said alkyl may contain ether groups.

In an embodiment, A and B independently of each other are: 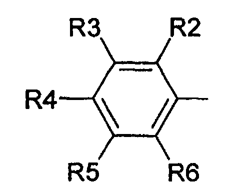 or B is H or C₁₋₃-alkyl, said alkyl may contain hydroxy, ester or ether groups (e.g. wherein the ether oxygen is directly attached to A-N(OH)C=O-, thus including N-hydroxy carbamic acid ester derivatives), and R2, R3, R4, R5 and R6 independently of each other are H, OH, NH₂, COOH, SO₃H, C₁₋₃-alkyl, acyl, NO₂, CN, Cl, Br, F, CF₃, NOH-CO-phenyl, CO-NOH-phenyl, COR12, OR7, NR8R9, COOR10, or NOH-CO-R11, wherein R7, R8 and R9 are C₁₋₃-alkyl or acyl, and R10, R11 and R12 are C₁₋₃-alkyl.

In another embodiment, A and B independently of each other are: 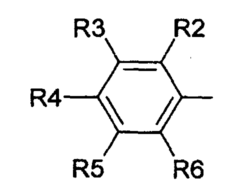 or B is H or C₁₋₃-alkyl, said alkyl may contain hydroxy or ether groups (e.g. wherein the ether oxygen is directly attached to A-N(OH)C=O-, thus including N-hydroxy carbamic acid ester derivatives), and R2, R3, R4, R5 and R6 independently of each other are H, OH, NH₂, COOH, SO₃H, CH₃, acyl, NO₂, CN, Cl, Br, F, CF₃, CO-NOH-phenyl, COCH₃, OR7, NR8R9, or COOCH₃, wherein R7, R8 and R9 are CH₃ or COCH₃.

In another embodiment, A and B independently of each other are: 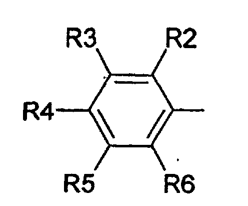 or B is H or C₁₋₃-alkyl, said alkyl may contain hydroxy or ether groups (e.g. wherein the ether oxygen is directly attached to A-N(OH)C=O-, thus including N-hydroxy carbamic acid ester derivatives), and R2, R3, R4, R5 and R6 independently of each other are H, OH, COOH, SO₃H, CH₃, acyl, NO₂, CN, Cl, Br, F, CO-NOH-phenyl, OCH₃, COCH₃, or COOCH₃.

In another embodiment, A and B independently of each other are: 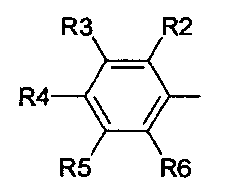 or B is C₁₋₃-alkyl, said alkyl may contain ether groups (e.g. wherein the ether oxygen is directly attached to A-N(OH)C=O-, thus including N-hydroxy carbamic acid ester derivatives), and R2, R3, R4, R5 and R6 independently of each other are H, OH, COOH, SO₃H, CH₃, NO₂, CN, Cl, Br, CO-NOH-phenyl, or OCH₃.

The terms "C₁₋ₙ-alkyl" wherein n can be from 2 through 16, as used herein, represent a branched or straight alkyl group having from one to the specified number of carbon atoms. Typical C₁₋₆-alkyl groups include, but are not limited to, methyl, ethyl, n-propyl, iso-propyl, butyl, iso-butyl, sec-butyl, tert-butyl, pentyl, iso-pentyl, hexyl, iso-hexyl and the like.

The term "acyl" as used herein refers to a monovalent substituent comprising a C₁₋₆-alkyl group linked through a carbonyl group; such as e.g. acetyl, propionyl, butyryl, isobutyryl, pivaloyl, valeryl, and the like.

In an embodiment at least one of the substituents R2, R3, R4, R5 and R6 of A are H, preferably at least two of the substituents R2, R3, R4, R5 and R6 of A are H, more preferably at least three of the substituents R2, R3, R4, R5 and R6 of A are H, most preferably at least four of the substituents R2, R3, R4, R5 and R6 of A are H, in particular all of R2, R3, R4, R5 and R6 of A are H.

In another embodiment at least one of the substituents R2, R3, R4, R5 and R6 of B are H, preferably at least two of the substituents R2, R3, R4, R5 and R6 of B are H, more preferably at least three of the substituents R2, R3, R4, R5 and R6 of B are H, most preferably at least four of the substituents R2, R3, R4, R5 and R6 of B are H, in particular all of R2, R3, R4, R5 and R6 of B are H.

In particular embodiments according to the invention the enhancing agent is selected from the group consisting of
4-nitrobenzoic acid-N-hydroxyanilide;
4-methoxybenzoic acid-N-hydroxyanilide;
N,N'-dihydroxy-N,N'-diphenylterephthalamide;
decanoic acid-N-hydroxyanilide;
N-hydroxy-4-cyanoacetanilide;
N-hydroxy-4-acetylacetanilide;
N-hydroxy-4-hydroxyacetanilide;
N-hydroxy-3-(N'-hydroxyacetamide)acetanilide;
4-cyanobenzoic acid-N-hydroxyanilide;
N-hydroxy-4-nitroacetanilide;
N-hydroxyacetanilide;
N-hydroxy-N-phenyl-carbamic acid isopropyl ester;
N-hydroxy-N-phenyl-carbamic acid methyl ester;
N-hydroxy-N-phenyl-carbamic acid phenyl ester;
N-hydroxy-N-phenyl-carbamic acid ethyl ester; and
N-hydroxy-N-(4-cyanophenyl)-carbamic acid methyl ester.

The enhancing agent of the invention may be present in concentrations of from 1 to 1000 µM, preferably of from 5 to 500 µM, and more preferably from 10 to 200 µM.

### Preparation of Enhancing Agents

The enhancing agents described in the present application may be prepared using methods well known to those skilled in the art; some of the enhancing agents are also commercially available.

The used compounds were prepared according to a general procedure described for N-hydroxyacetanilide (see Organic Syntheses 67, 1989, p. 187-192) followed by standard purification procedures.

### Oxygen

Molecular oxygen from the atmosphere will usually be present in sufficient quantity. If more O₂ is needed, additional oxygen may be added.

### Enzyme

In the context of the present invention the enzyme may be a laccase or a laccase related enzyme.

The enzyme of the invention may typically be present in concentrations of from 1 to 10000 µg enzyme protein per liter aqueous solution, preferably of from 5 to 2000 µg enzyme protein per liter aqueous solution, more preferably of from 5 to 1000 µg enzyme protein per liter aqueous solution, and most preferably of from 1 to 500 µg enzyme protein per liter aqueous solution.

### Laccases and Laccase Related Enzymes

In the context of this invention, laccases and laccase related enzymes comprise any laccase enzyme comprised by the enzyme classification (EC 1.10.3.2), any catechol oxidase enzyme comprised by the enzyme classification (EC 1.10.3.1), any bilirubin oxidase enzyme comprised by the enzyme classification (EC 1.3.3.5) or any monophenol monooxygenase enzyme comprised by the enzyme classification (EC 1.14.18.1).

The above mentioned enzymes may be microbial, i.e. derived from bacteria or fungi (including filamentous fungi and yeasts), or they may be derived from plants.

Suitable examples from fungi include a laccase derivable from a strain of *Aspergillus, Neurospora,* e.g., *N. crassa, Podospora, Botrytis, Collybia, Fomes, Lentinus, Pleurotus, Trametes,* e.g., *T. villosa* and *T. versicolor, Rhizoctonia,* e.g., *R. solani, Coprinus,* e.g., *C. cinereus, C. comatus, C. friesii,* and C. *plicatilis, Psathyrella,* e.g., *P. condelleana, Panaeolus,* e.g., *P. papilionaceus, Myceliophthora,* e.g., *M. thermophila, Schytalidium,* e.g., *S. thermophilum, Polyporus,* e.g., *P. pinsitus, Pycnoporus,* e.g. *P. cinnabarinus, Phlebia,* e.g., *P. radita* (WO 92/01046), *or Coriolus,* e.g., *C. hirsutus* (JP 2-238885).

Suitable examples from bacteria include a laccase derivable from a strain of *Bacillus.*

A laccase derived from *Coprinus, Myceliophthora, Polyporus, Pycnoporus, Scytalidium* or *Rhizoctonia* is preferred; in particular a laccase derived from *Coprinus cinereus, Myceliophthora thermophila, Polyporus pinsitus, Pycnoporus cinnabarinus, Scytalidium thermophilum* or *Rhizoctonia solani.*

The laccase or the laccase related enzyme may furthermore be one which is producible by a method comprising cultivating a host cell transformed with a recombinant DNA vector which carries a DNA sequence encoding said laccase as well as DNA sequences encoding functions permitting the expression of the DNA sequence encoding the laccase, in a culture medium under conditions permitting the expression of the laccase enzyme, and recovering the laccase from the culture.

### Determination of Laccase Activity (LACU)

Laccase activity is determined from the oxidation of syringaldazin under aerobic conditions. The violet colour produced is photometered at 530 nm. The analytical conditions are 19 mM syringaldazin, 23 mM acetate buffer, pH 5.5, 30°C, 1 min. reaction time.

1 laccase unit (LACU) is the amount of enzyme that catalyses the conversion of 1.0 µmole syringaldazin per minute at these conditions.

### Determination of Laccase Activity (LAMU)

Laccase activity is determined from the oxidation of syringaldazin under aerobic conditions. The violet colour produced is photometered at 530 nm. The analytical conditions are 19 mM syringaldazin, 23 mM Tris/maleate buffer, pH 7.5, 30°C, 1 min. reaction time.

1 laccase unit (LAMU) is the amount of enzyme that catalyses the conversion of 1.0 µmole syringaldazin per minute at these conditions.

### The composition

The antimicrobial composition according to the invention may be formulated as a solid or a liquid.

When formulated as a liquid, the composition is typically an aqueous composition.

When formulated as a solid, the composition is typically a powder, a granulate, a paste or a gelled product.

It is preferred to use a two part formulation system in the cases where hydrogen peroxide is needed, whereby the hydrogen peroxide is separate from the other components.

The composition of the invention may further comprise auxiliary agents such as wetting agents, thickening agents, buffer, stabilisers, perfume, colourants, fillers and the like.

Useful wetting agents are surfactants, i.e., non-ionic, anionic, amphoteric or zwitterionic surfactants.

The composition of the invention may be a concentrated product or a ready-to-use product. In use, the concentrated product is typically diluted with water to provide a medium having an effective antimicrobial activity, applied to the object to be disinfected or preserved, and allowed to react with the micro-organisms present.

The optimum pH of such an aqueous composition is usually a compromise between the optimum stability and optimum activity of the enzyme in question. In one aspect of the invention pH is in the range of pH 3 to 10.5, and in another aspect of the invention pH is in the range of pH 5 to 9.

### The method

The present invention also provides a method for killing or inhibiting microbial cells comprising treating said microbial cells with the composition of the invention. Said treatment may be carried out with an effective amount of said composition.

As an "effective amount" is meant an amount suitable for obtaining the required antimicrobial effect in the chosen application; e.g. to reduce the number of living cells to 10%, 1% or less than 1%; or to prevent the number of living cells from doubling during 12 hours, 1 day, 5 days, 30 days or more than 30 days.

### Uses

The composition of the invention may be incorporated into a detergent or cleaning composition typically comprising other enzyme types as well (see below).

The composition of the invention can also be used for inhibiting micro-organisms present in laundry, by treating the laundry with a soaking, washing or rinsing liquor comprising an effective amount of the composition.

When used for preservation of paint, food, beverages, cosmetics such as lotions (e.g. eye lotions), liquids, creams, gels, pastes, ointments (e.g. eye ointments), soaps, shampoos, conditioners, antiperspirants, deodorants, mouth wash, nasal sprays, contact lens products, enzyme formulations, or food ingredients, the composition used in the method of the present invention may be incorporated into e.g. water based paint, unpreserved food, beverages, cosmetics, contact lens products, food ingredients or anti-inflammatory product in an amount effective for killing or inhibiting growth of microbial cells.

In particular, the composition of the invention may be used as a preservation agent or a disinfection agent in water based paints (see below).

Furthermore, the composition according to the present invention may by useful as a disinfectant, e.g., in the treatment of acne, infections in the eye or the mouth, skin infections; in antiperspirants or deodorants; in foot bath salts; for cleaning and disinfection of contact lenses, hard surfaces, teeth (oral care), wounds, bruises and the like.

In general the composition of the present invention is useful for cleaning, disinfecting or inhibiting microbial growth on any hard surface. Examples of surfaces, which may advantageously be contacted with the composition of the invention are surfaces of process equipment used, e.g., in dairies, chemical or pharmaceutical process plants, water sanitation systems, paper pulp processing plants, water treatment plants, and cooling towers. The composition of the invention may be used in an amount, which is effective for cleaning, disinfecting or inhibiting microbial growth on the surface in question.

In particular, the composition of the invention may be used for disinfecting and inhibiting microbial growth in paper and pulp processing plants.

Further, it is contemplated that the composition of the invention can advantageously be used in a cleaning-in-place (C.I.P.) system for cleaning of process equipment of any kind.

The method of the invention may additionally be used for cleaning surfaces and cooking utensils in food processing plants and in any area in which food is prepared or served such as hospitals, nursing homes, restaurants, especially fast food restaurants, delicatessens and the like. It may also be used as an antimicrobial in food products and would be especially useful as a surface antimicrobial for cheese, fruits and vegetables and for food in salad bars.

The composition of the present invention is also useful for microbial control of water lines, and for disinfection of water, in particular for disinfection of industrial water.

### Conservation/preservation of paints

Conservation of paint products in cans has in the art been accomplished by adding non-enzymatic organic biocides to the paints. In the context of the invention paint is construed as a substance comprising a solid coloring matter dissolved or dispersed in a liquid vehicle such as water, organic solvent and/or oils, which when spread over a surface, dries to leave a thin colored, decorative and/or protective coating. Typically isothiazoliones, such as 5-chlor-2-methyl-4-thiazoli-3-on, has been added to the paint as biocides to inhibit/prevent microbial growth in the paint. The method of the invention can however suitably be applied in this field, thereby solving the problem of the ever present environmental bio-hazards of using toxic organic biocides by replacing these toxic biocides with environmentally compatible enzymes. Thus the present invention provides a method for conservation of a paint comprising contacting said paint with a phenol oxidizing enzyme and an enhancing agent according to the invention. Further the invention provides a paint composition comprising a phenol oxidizing enzyme and an enhancing agent as described in the present invention.

The paint is preferably a water based paint, *i.e*. the solids of the paint is dispersed in an aqueous solution. The paint may contain 0-20 % organic solvent, preferable 0-10%, e.g. 0-5%.

The enzyme may be added to the paint in an amount of 0.0001-100 mg active enzyme protein per litre paint, preferably 0.001-10 mg/l, e.g. 0.01-5 mg/l, while the enhancing agent may be added in an amount of 10-500 µM, preferably 25-250 µM, e.g. 100 µM of the paint composition.

### Detergent Compositions

The antimicrobial composition of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the antimicrobial composition of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.
Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase™, Savinase™, Primase™, Duralase™, Esperase™, and Kannase™ (Novo Nordisk A/S), Maxatase™, Maxacal™, Maxapem™, Properase™, Purafect™, Purafect OxP™, FN2™, and FN3™ (Genencor International Inc.).
Lipases: Suitable lipases include those of bacterial or fungal origin. Chemically modified or protein engineered mutants are included. Examples of useful lipases include lipases from *Humicola* (synonym *Thermomyces),* e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), P. *fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase™ and Lipolase Ultra™ (Novo Nordisk A/S).
Amylases: Suitable amylases (α and/or β) include those of bacterial or fungal origin. Chemically modified or protein engineered mutants are included. Amylases include, for example, α-amylases obtained from *Bacillus,* e.g. a special strain of *B. licheniformis*, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novo Nordisk A/S), Rapidase™ and Purastar™ (from Genencor International Inc.).
Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novo Nordisk A/S), Clazinase™, and Puradax HA™ (Genencor International Inc.), and KAC-500(B)™ (Kao Corporation).
Peroxidases/Oxidases: Suitable peroxidases/oxidases include those of plant, bacterial or fungal origin. Chemically modified or protein engineered mutants are included. Examples of useful peroxidases include peroxidases from *Coprinus*, e.g. from *C. cinereus,* and variants thereof as those described in WO 93/24618, WO 95/10602, and WO 98/15257.

Commercially available peroxidases include Guardzyme™ (Novo Nordisk A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or nonaqueous.

The detergent composition comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

The present invention is further illustrated in the following examples which are not in any way intended to limit the scope of the invention as claimed.

### EXAMPLE 1

### Antibacterial activity of Coprinus peroxidase with N-hydroxyacetanilide as electron donor.

The antimicrobial activity of recombinant *Coprinus cinereus* peroxidase (rCiP), obtained as described in WO 92/16634, at pH 6 and pH 8 by use of N-hydroxyacetanilide as electron donor was tested.

Antimicrobial activity was evaluated in 0.05 M MES-buffer (Sigma M8250) (pH 6) or 0.05 HEPES-buffer (Sigma H3375) (pH 8); buffers were sterilised by filter sterilisation.

The antimicrobial activity was determined against *Pseudomonas fluorescens* (Gram et al. 1990, International Journal of Food Microbiology 10: 303-316) and *Staphylococcus epidermidis* (DSM 20042). Cells were grown in Tryptone Soya Broth (Oxoid CM129) at 30°C *(S. epidermidis)* or 25°C (P. *fluorescens*) for 24 h and diluted in the MES-buffer to a final cell concentration of approximately 10⁶ cfu/ml. The cell suspensions were mixed with rCiP (3 POXU/ml), enhancing agent (0.2 mM) and hydrogen peroxide (0.5 mM) for 20 min at 40°C. The bactericidal activity was determined by incubation in Malthus. Detection times measured by the Malthus instrument were converted to cfu/ml by a calibration curve. Either direct or Indirect Malthus measurements were used when enumerating total survival cells (Malthus Flexi M2060, Malthus Instrument Limited). By the direct measurements, the cell metabolism was determined by conductance measurements in the growth substrate. By the indirect measurements, 3 ml of growth medium was transferred to the outer chamber of the indirect Malthus cells, and 0.5 ml of sterile KOH (0.1 M) was transferred to the inner chamber. The cell suspensions were after enzyme treatment transferred to the outer chamber of the Malthus cell. As cells are growing in the outer chamber they produce CO₂ which will dissolve in the KOH in the inner chamber and thereby change the conductance of the KOH. The amount of CO₂ formed by the respiring cells surviving the enzyme treatment was used for estimating the number of viable cells. When the conductance change is measurable by the Malthus, a detection time (dt) will be recorded. The dt's were converted to colony counts by use of a calibration curve relating cfu/ml to dt (Johansen et al. 1995. Journal of Applied Bacteriology 78:297-303, Johansen et al. 1997, Applied and Environmental Microbiology 63:3724-3728).

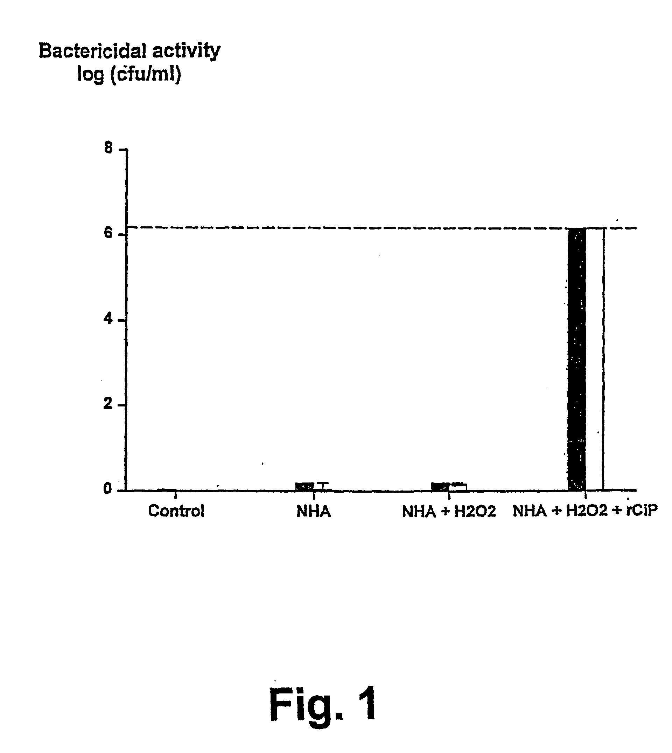

No bactericidal activity of N-hydroxyacetanilide or N-hydroxyacetanilide combined with hydrogen peroxide was observed. Whereas a total kill was obtained by the combined system; N-hydroxyacetanilide, hydrogen peroxide and rCiP (see Fig 1.). Gram-negative bacteria are in general most resistant towards combined oxidoreductase and enhancing agent systems. However, the total kill, corresponding to a cell reduction of approximately 10⁶ CFU/ml (Colony Forming Units), was obtained at both pH 6 and 8 (Fig. 1) against the Gram-negative bacterium *P. fluorescens.*

### EXAMPLE 2

### Bactericidal activity of laccases and N-hydroxyacetanilide

Antibacterial activity of *Polyporus pinsitus* laccase (rPpL), obtained as described in WO 96/00290), and *Coprinus cinereus* laccase (rCcL), obtained as described in WO 97/08325, was determined with N-hydroxyacetanilide as enhancing agent against *Pseudomonas aeruginosa* (ATCC 10145), *Enterobacter aerogenes* (ATCC 13048) and *Enterococcus faecalis* (DSM 2570). The bactericidal activity was determined as described in Example 1, the antimicrobial activity of rPpL (1 mg/L) was evaluated at pH 6, whereas rCcL (1 mg/L) was evaluated at pH 8.

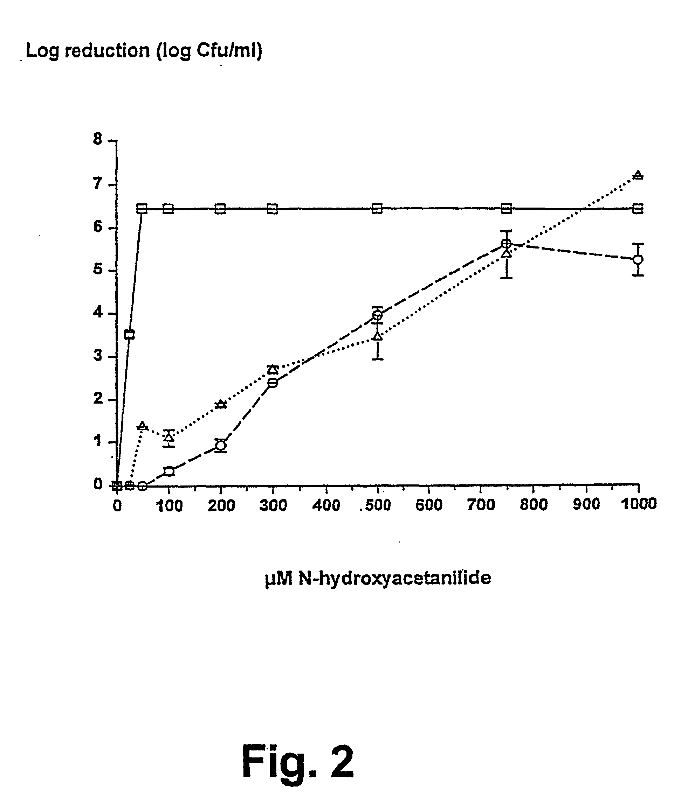

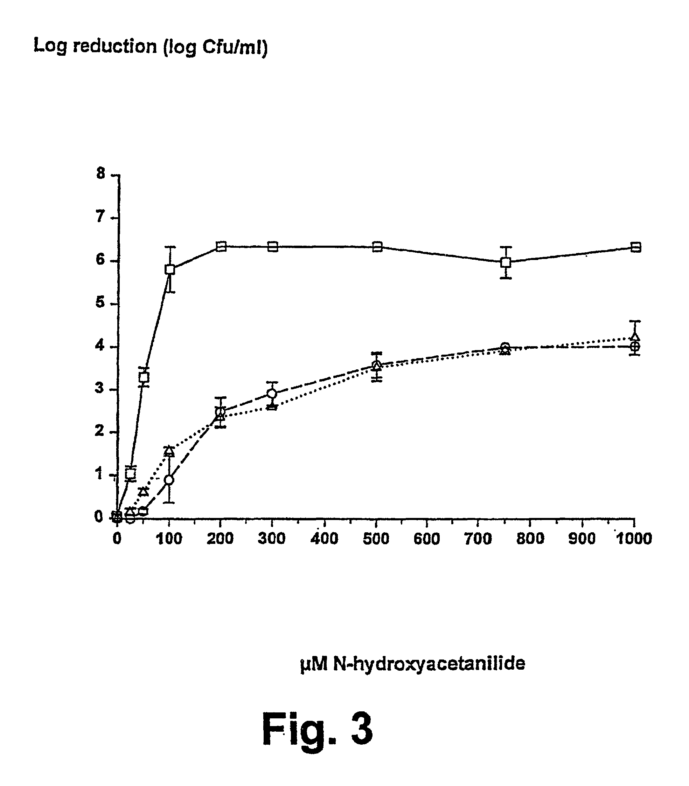

Dose-Response curves for N-hydroxyacetanilide are shown when combined with rPpL (Fig. 2) or rCcL (Fig. 3). A significant bactericidal activity was obtained against all the test organisms, with the Gram-negative strains being the most resistant. Bactericidal activity against *P. aeruginosa* ATCC 10145 was obtained at N-hydroxyacetanilide concentrations above 200 µM. When using the rPpL at pH 6, a total kill of the two gram-negative bacteria was obtained at high concentration of N-hydroxyacetanilide.

### EXAMPLE 3

### Bactericidal activity of various phenol oxidizing enzymes and enhancing agents

Antibacterial activity of *Coprinus cinereus* peroxidase (rCiP), *Polyporus pinsitus* laccase (rPpL), *Coprinus cinereus* laccase (rCcL) and *Rhizoctonia solani* laccase (rRsL) (as described in WO 95/07988) was determined with different enhancing agents at pH 6 and 8 (buffers; see Example 1). The rCiP was combined with 0.5 mM hydrogen peroxide.

Antimicrobial activity of rCiP and rPpL was determined against Pseudomonas fluorescens (Gram et al. 1990, International Journal of Food Microbiology 10:303-316), whereas antimicrobial activity of rCcL and rRsL was determined against *Pseudomonas aeruginosa* (ATCC 10145).

The bactericidal activity was determined as described in example 1. The different combinations and the results are shown in Table 1. The results are shown as the reduction in living cell number. Thus, a reduction of 4 correspond to a 4 log reduction of living cells e.g. from 10⁶ CFU/ml to 10² CFU/ml.

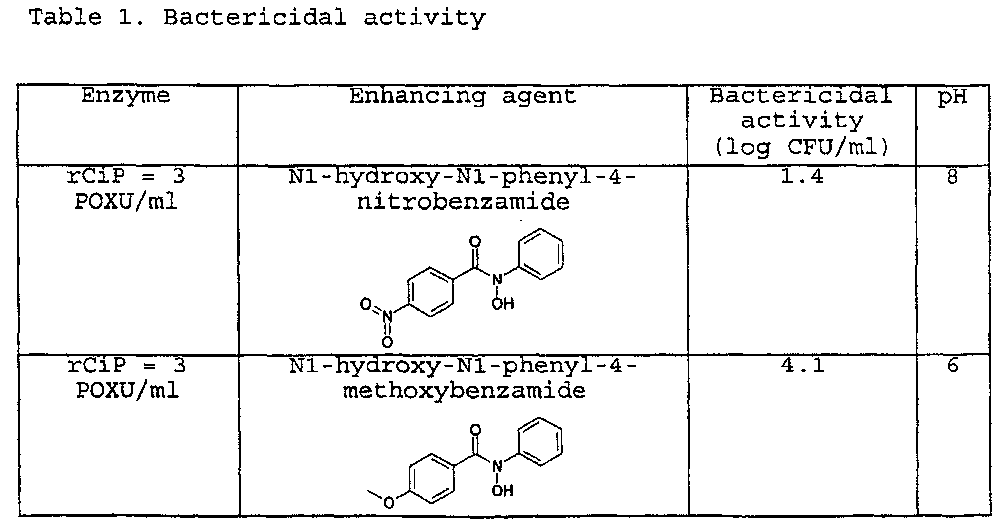

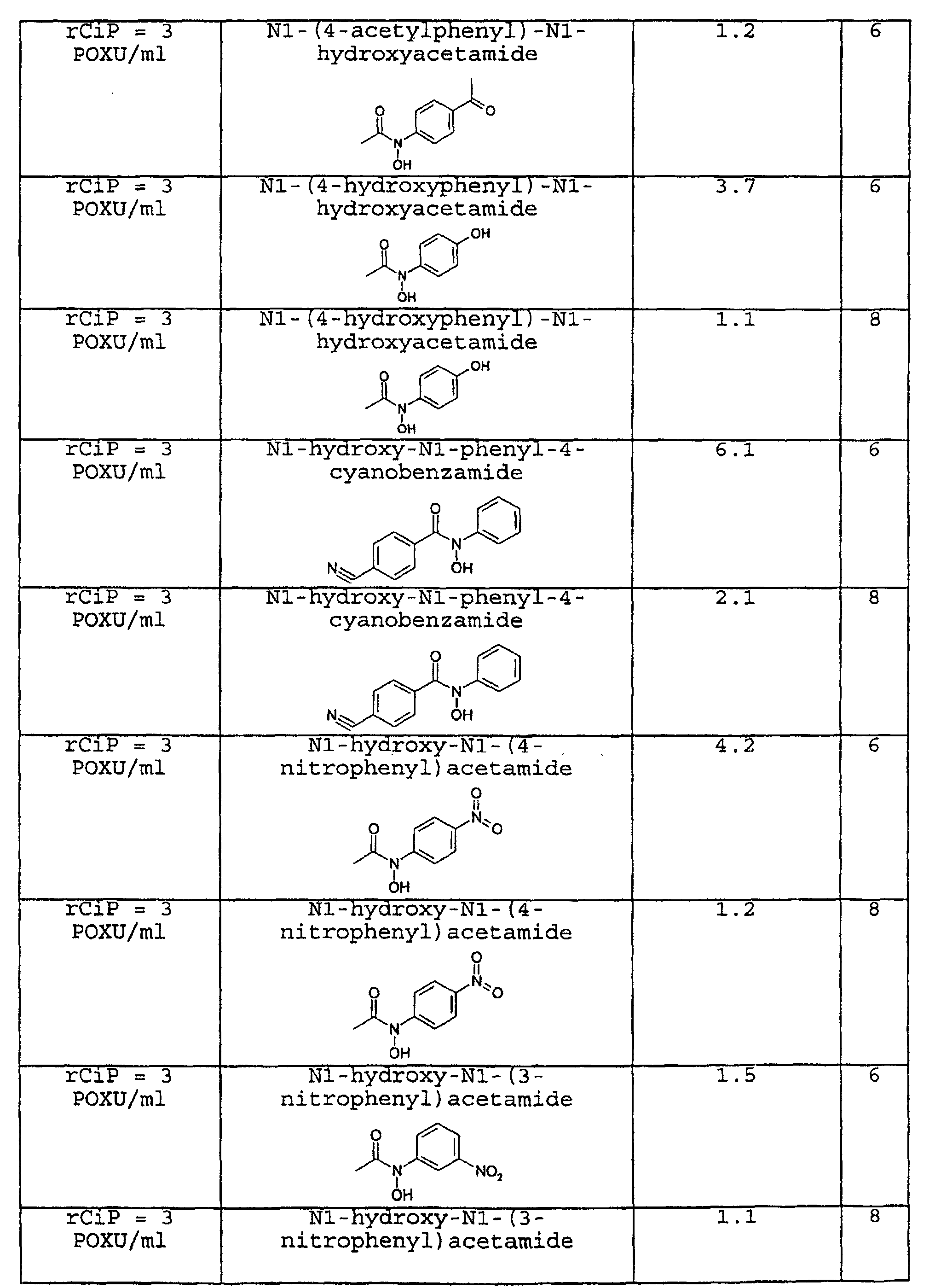

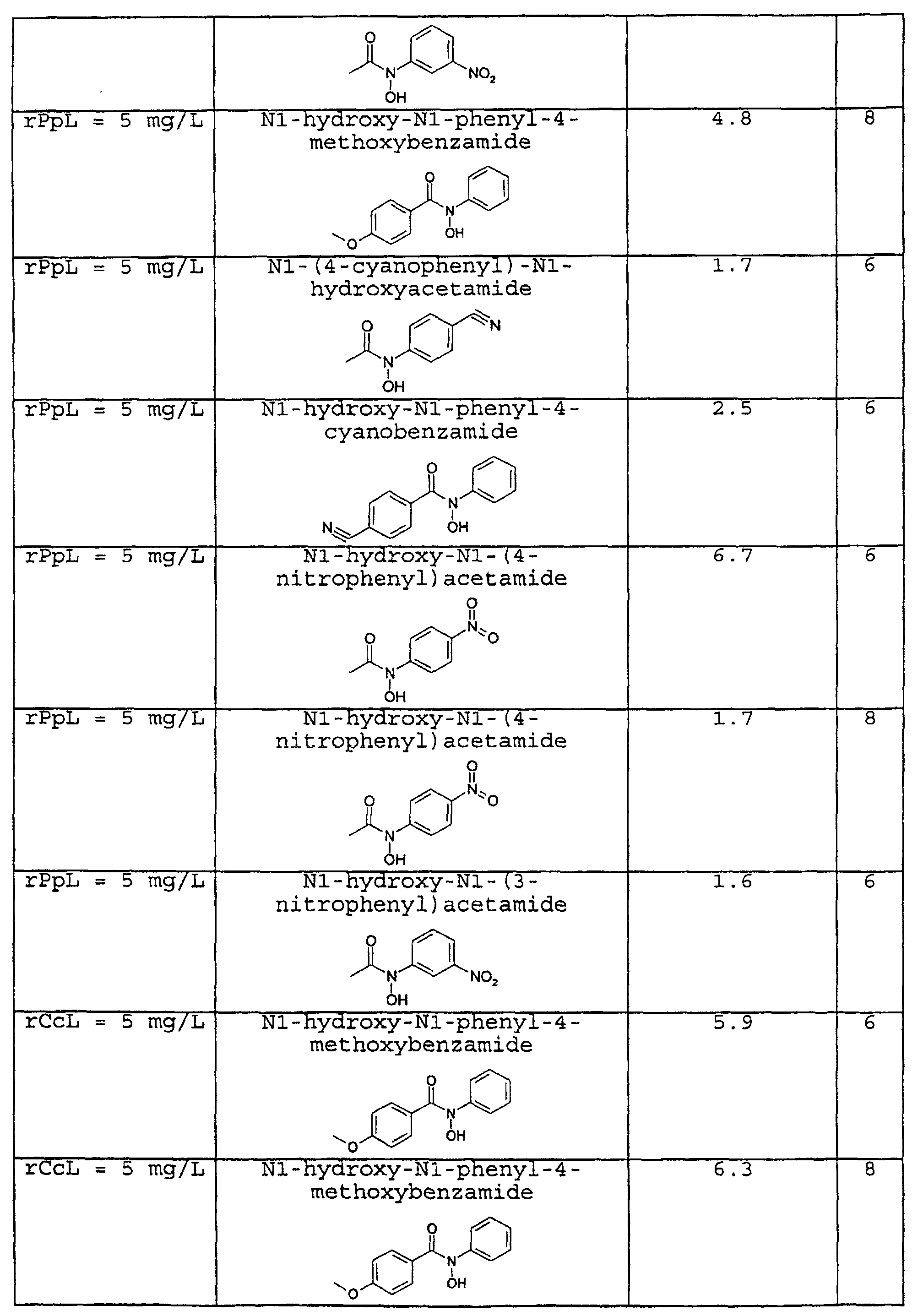

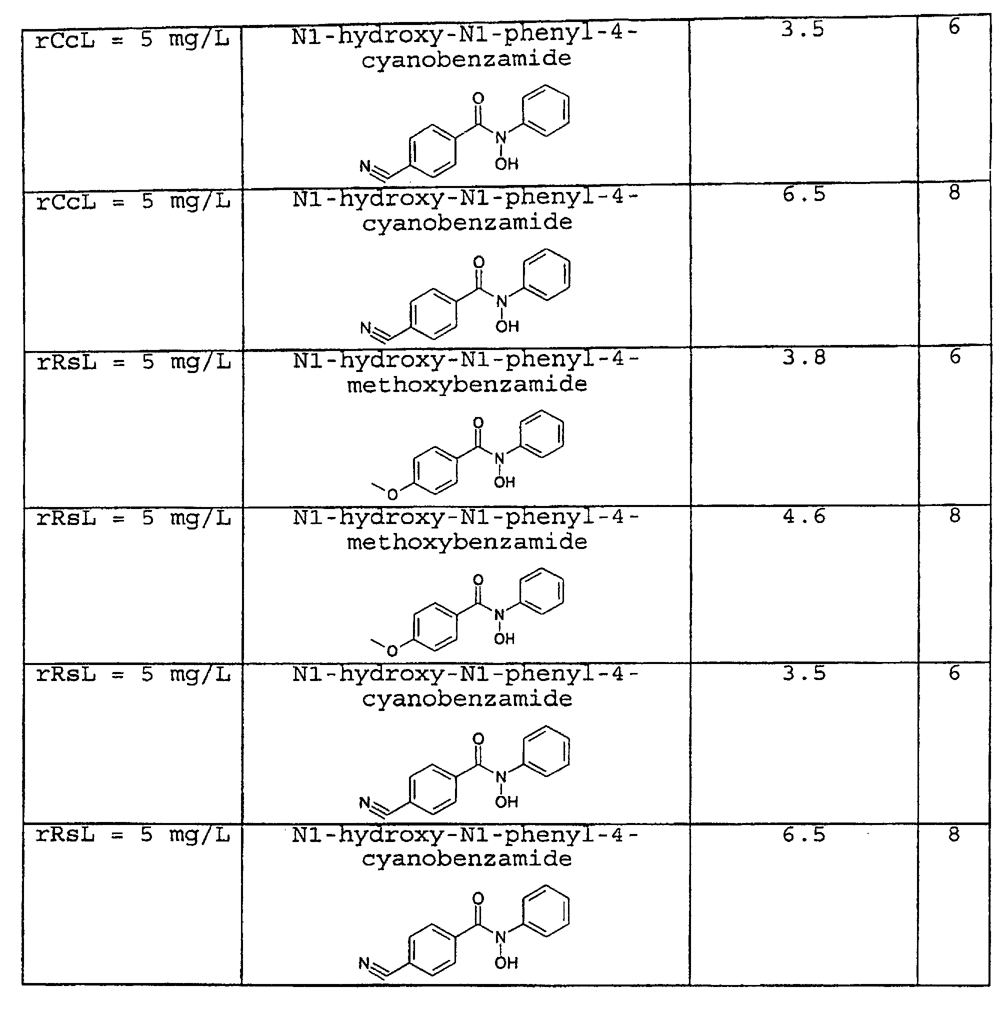

### EXAMPLE 4

### Examples of enhancing agents

A) *N*1-hydroxy-*N*1-phenyl-4-nitrobenzamide,
   4-nitrobenzoic acid-*N*-hydroxyanilide(CAS 2029-61-0); 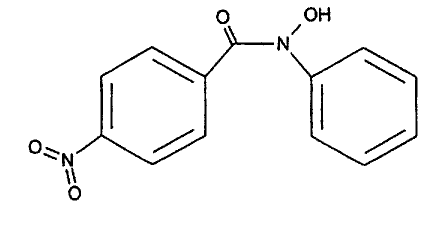
B) *N*1-hydroxy-*N*1-phenyl-4-methoxybenzamide,
   4-methoxybenzoic acid-*N*-hydroxyanilide (CAS 13664-49-8) ; 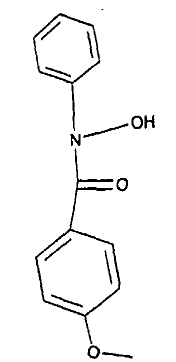
C) *N,N'*-dihydroxy-*N,N'*-diphenylterephthalamide (CAS 61494-26-6) ; 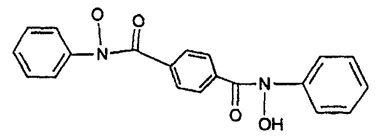
D) *N*1-(4-cyanophenyl)-*N*1-hydroxyacetamide,
   N-hydroxy-4-cyanoacetanilide (CAS 80584-65-2); 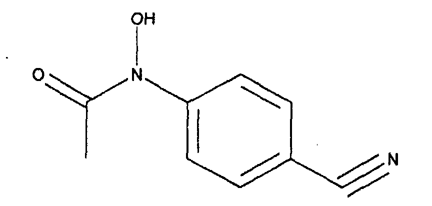
E) *N*1-hydroxy-*N*1-phenyldecaneamide,
   decanoic acid-*N*-hydroxyanilide (CAS 25310-16-1); 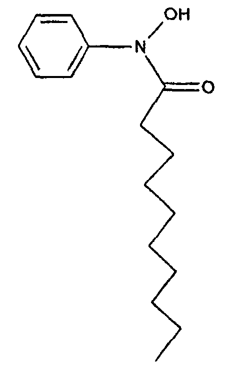
F) *N*1-hydroxy-*N*1-phenyl-4-cyanobenzamide,
   4-cyanobenzoic acid-*N*-hydroxyanilide; 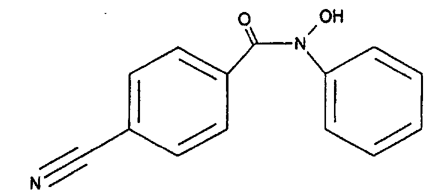
G) *N*1-(4-acetylphenyl)-*N*1-hydroxyacetamide,
   N-hydroxy-4-acetylacetanilide (CAS 67274-51-5); 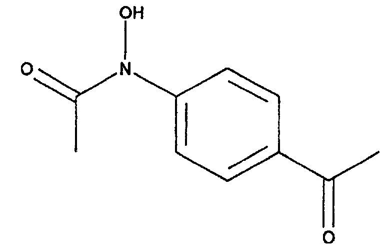
H) *N*1-hydroxy-*N*1-(3-nitrophenyl)acetamide; 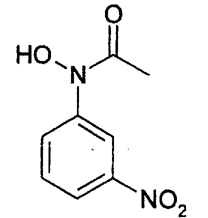
I) *N*1-(4-hydroxyphenyl)-*N*1-hydroxyacetamide,
   *N*-hydroxy-4-hydroxyacetanilide (CAS 63975-21-3); 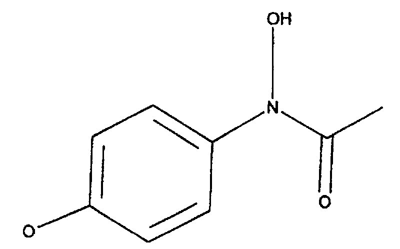
K) *N*1-hydroxy-*N*1-(4-nitrophenyl)acetamide,
   N-hydroxy-4-nitroacetanilide (CAS 67274-52-6); 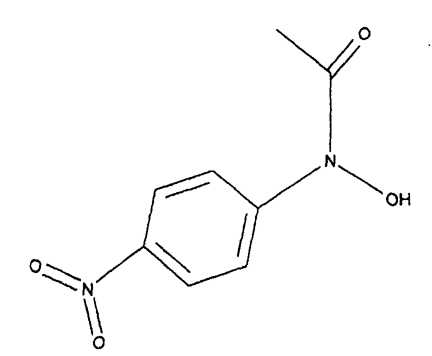
L) *N*-hydroxyacetanilide (CAS 1795-83-1); 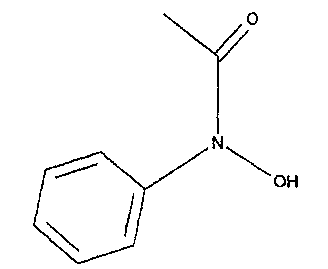
M) *N*-hydroxy-*N*-phenyl-carbamic acid isopropyl ester (CAS 4279-16-7); 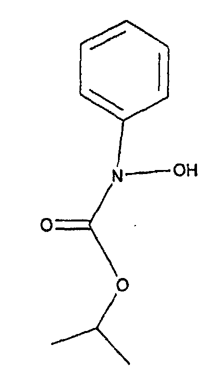
N) *N*-hydroxy-*N*-phenyl-carbamic acid methyl ester (CAS 28091-62-5); 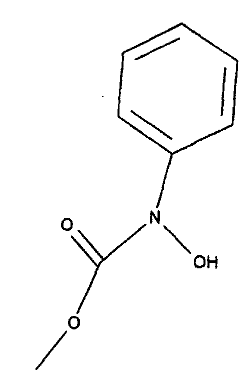
O) *N*-hydroxy-*N*-phenyl-carbamic acid phenyl ester (CAS 4645-72-1); 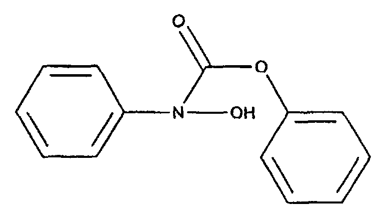
P) *N*-hydroxy-*N*-phenyl-carbamic acid ethyl ester (CAS 18631-99-7); 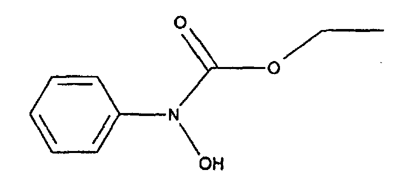
Q) *N*-hydroxy-*N*-(4-cyanophenyl)-carbamic acid methyl ester

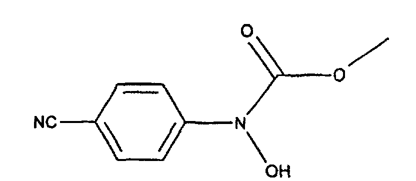

All compounds were synthesized according to a general procedure as outlined in Organic Syntheses **1989**, 67, 187-192 for the synthesis of compound L). The procedure was modified concerning the work-up of the reaction mixtures and the purification of products. All structures were verified by their melting points from the literature, if available, and by ¹H-NMR on VARIAN Mercury 400.

The following compounds have been described earlier:
A): S. Horner, Justus Liebigs Ann. Chem. 606, 1957, 24-28; O. Neunhoeffner, R. Gottschlich, Justus Liebigs Ann. Chem. 736, 1970, 100-109.
B): S. Horner, Justus Liebigs Ann. Chem. 606, 1957, 24-28; S. Jaimins, J. Indian Chem. Soc. 47, 1970, 247-249.
C): N.R. Gandhi, K.N. Munshi, J. Indian Chem. Soc. 59, 11112, 1982, 1290-1295; Munshi, K.N., Kharche, V.W., Junejai, H.D., Indian J. Chem. Sect. A, 27, 3, 1988, 222-224.
D): C. Poth Brink, A.C. Crumbliss, J. Org. Chem., 47, 7, 1982, 1171-1176.
E): Gupta; Tandon, J.Indian Chem.Soc., 46, 1969, 831-833.
G): C. Poth Brink, A.C. Crumbliss, J. Org. Chem., 47, 7, 1982, 1171-1176.
I): Healey; Calder, Aust.J.Chem., 32, 1979, 1307-1315; Gemborys,M.B. et al., J.Med.Chem., 21, 1978, 649-652.
K): Matlin,S.A. et al., J.Chem.Soc.Perkin Trans.1, 1979, 2481-2487.
M): Baskakow; Mel'nikow, Khim.Nauka Prom-st., 3, 1958, 683, Chem.Abstr., 1959, 7062; Patent, BASF-A.G., FR 1507608, 1967, Chem.Abstr., EN, 70, 57386e, 1969; Baskakow et al.,
   Biol.Akt.Soedin.1968, 1968, 244-49.
N): Faddeewa; Baskakow, Biol.Akt.Soedin.1968, 1968, 199-06.
O): Oesper; Broker, J.Amer.Chem.Soc., 47, 1925, 2608; Baskakov et al., J.Org.Chem.USSR (Engl.Transl.), 3, 1967, 108, Zh.Org.Khim., 3, 1967, 112; Baskakow et al., J.Org.Chem.USSR (Engl.Transl.), 3, 1967, 108, Zh.Org.Khim., 3, 1967, 112.
P): Bamberger, Chem.Ber., 52, 1919-120; Journal, Tisue et al.,Tetrahedron, 24, 1968, 999-004.

### Synthesis of compounds F), H), and Q) are described below:

### Synthesis of N1-hydroxy-N1-phenyl-4-cyanobenzamide (F)

A solution of *N*-Phenylhydroxylamine (1.77g, 16.2 mmol) in THF (30ml) was synthesized as outlined in Org. Synth. **1989,** 67, 187-192. A slurry of sodium bicarbonate (2g in 2 ml water) was added to the solution. The mixture was cooled to -2°C and 4-cyanobenzoyl chloride (3.23g, 19.51mmol) was added dropwise. Stirring was continued overnight, before water (200 ml) was added. The reaction mixture was extracted 3x with methylene chloride. The solvent of the combined organic phases was removed *in vacuo.* The residue was purified chromatography using a Biotage Flash40i with SIM fitted with a 4.0x15.0cm cartridge using AcOEt/Heptane (3:5, v:v) as eluting agent to give 0.55 g of pure f) (14%): mp: 146-147°C. ¹H NMR (400MHz, DMSO) d 10.93 (s, 1H), 7.93 (d, 2H), 7.80 (d, 2H), 7.62 (d, 2H), 7.42 (dd, 2H), 7.24 (t, 1H). ¹³C NMR (100 MHz, DMSO) d 165.91, 140.82, 139.59, 131.45, 128.38, 128.11, 125.43, 121.30, 117.85, 111.99. Anal. Calculated for C₁₄H₁₀N₂O₂: C, 70.58; H, 4.23; N, 11.76 Found: C, 70.62; H, 4.32; N, 11.64.

### Synthesis of N1-hydroxy-N1-(3-nitrophenyl)acetamide (H)

Hydrazine hydrate (3.56g, 71.11mmol) was added dropwise to a suspension of 1,3-dinitrobenzene (6.0g, 35.69mmol) and wet 5% rhodium carbon (120mg) in tetrahydrofuran (30ml) keeping the temperature below 15°C. After stirring at room temperature overnight, the mixture is filtered and the catalyst washed with a little tetrahydrofuran. A slurry of sodium bicarbonate (6g in 5 ml water) was added to the solution. The mixture was cooled to 0°C and acetyl chloride (6.17g, 78.60mmol) was added dropwise. Stirring was continued overnight, followed by the addition of a solution of sodium hydroxide (5g in 100 ml). The aqueous phase was separated, petroleum ether was added, and the tetrahydrofuran phase was separated again. The combined organic phases were extracted with aqueous 10% sodium hydroxide solution. The combined aqueous phases were washed with CH₂Cl₂ and then neutralized with hydrochloric acid. The mixture was extracted with CH₂Cl₂ and the extracts were combined, dried over magnesium sulphate, filtered and concentrated at reduced pressure. Upon the addition of petroleum ether, light orange crystals formed to give 4.59 g of pure (h) (66%): mp 92-94°C. ¹H NMR (400MHz, CDCl₃) d 9.41 (1H), 8.36 (1H), 7.96 (1H), 7.91 (1H), 7.46 (1H), 2.28 (3H). ¹³C NMR (100 MHz, CDCl3) d 149.01, 130.52, 127.42, 121.25, 116.43, 23.45. Calculated for C₈H₈N₂O₄: C, 48.98; H, 4.11; N, 14.28 Found: C, 49.55; H, 4.16; N, 14.21.

### Synthesis of N-hydroxy-N-(4-cyanophenyl)-carbamic acid methyl ester (Q)

A solution of *N*-4-(cyanophenyl)hydroxylamine (3.13g, 21.1 mmol) in THF (30ml) was synthesized following the procedure described in Org. Synth. 1989, 67, 187-192 for the preparation of *N*-4-phenylhydroxylamine. A slurry of sodium bicarbonate (4.6g in 7 ml water) was added to the solution. The mixture was cooled to -0°C and methyl chloroformate (1.43ml, 21.1mmol) was added dropwise. Stirring was continued overnight before NaOH (8%, 30 ml) was added over 45 min. The water phase was separated. Petroleum ether (60 ml) was added to the THF-phase and the water phase was separated again. The organic phase was extracted 2x with 8% NaOH (40ml). The combined water phases were washed with CH₂Cl₂ and neutralized with HCl (cooling!). The acidified water phase was extracted 3x with CH₂Cl₂ (60ml). The combined organic phases were dried over MgSO₄, filtered, and the solvent was removed *in vacuo* to give crude (q). The compound was recrystallized from toluene/heptane to give 2.69g of white crystals (66%): mp: 118-121°C. ¹H NMR (400MHz, CDCl₃) d 7.71 (d, 2H), 7.63 (d, 2H), 3.93 (s, 3H). ¹³C NMR (100 MHz, CDCl₃) d 154.16, 143.89, 132.52, 119.01, 118.54, 107.17, 54.22. Anal. Calculated for C₉H₈N₂O₃: C, 56.25; H, 4.20; *N,* 14.58.
Found: C, 55.51; H, 4.24; N, 14.73.

## Claims

1. A method for killing or inhibiting microbial cells comprising treating said microbial cells with a composition comprising a laccase or a laccase related enzyme together with oxygen and an enhancing agent of the following formula: 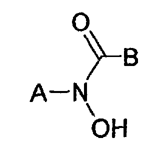 in which A and B independently of each other are: 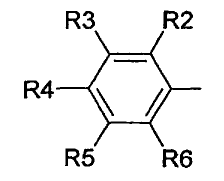 or B is H or C₁₋₁₆-alkyl, said alkyl may contain hydroxy, ester or ether groups, and R2, R3, R4, R5 and R6 independently of each other are H, OH, NH₂, COOH, SO₃H, C₁₋₁₂-alkyl, acyl, NO₂, CN, Cl, Br, F, CF₃, NOH-CO-phenyl, CO-NOH-phenyl, C₁₋₆-CO-NOH-A, CO-NOH-A, COR12, phenyl-CO-NOH-A, OR7, NR8R9, COOR10, or NOH-CO-R11, wherein R7, R8, R9, R10, R11 and R12 are C₁₋₁₂-alkyl or acyl.

2. The method according to claim 1, in which A and B of the enhancing agent independently of each other are: 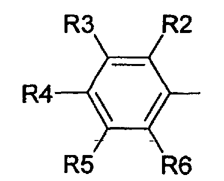 or B is H or C₁₋₃-alkyl, said alkyl may contain hydroxy, ester or ether groups, and R2, R3, R4, R5 and R6 independently of each other are H, OH, NH₂, COOH, SO₃H, C₁₋₃-alkyl, acyl, NO₂, CN, Cl, Br, F, CF₃, NOH-CO-phenyl, CO-NOH-phenyl, COR12, OR7, NR8R9, COOR10, or NOH-CO-R11, wherein R7, R8 and R9 are C₁₋₃-alkyl or acyl, and R10, R11 and R12 are C₁₋₃-alkyl.

3. The method according to claim 1, in which A and B of the enhancing agent independently of each other are: 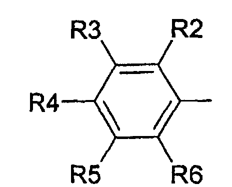 or B is H or C₁₋₃-alkyl, said alkyl may contain hydroxy or ether groups, and R2, R3, R4, R5 and R6 independently of each other are H, OH, NH₂, COOH, SO₃H, CH₃, acyl, NO₂, CN, Cl, Br, F, CF₃, CO-NOH-phenyl, COCH₃, OR7, NR8R9, or COOCH₃, wherein R7, R8 and R9 are CH₃ or COCH₃.

4. The method according to claim 1, in which A and B of the enhancing agent independently of each other are: 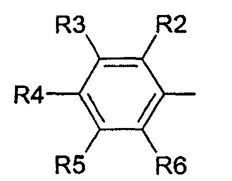 or B is H or C₁₋₃-alkyl, said alkyl may contain hydroxy or ether groups, and R2, R3, R4, R5 and R6 independently of each other are H, OH, COOH, SO₃H, CH₃, acyl, NO₂, CN, Cl, Br, F, CO-NOH-phenyl, OCH₃, COCH₃, or COOCH₃.

5. The method according to claim 1, in which A and B of the enhancing agent independently of each other are: 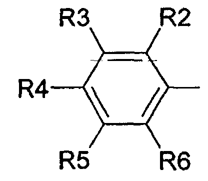 or B is C₁₋₃-alkyl, said alkyl may contain ether groups, and R2, R3, R4, R5 and R6 independently of each other are H, OH, COOH, SO₃H, CH₃, NO₂, CN, Cl, Br, CO-NOH-phenyl, or OCH₃.

6. The method according to claim 1, in which the enhancing agent is selected from the group consisting of
4-nitrobenzoic acid-N-hydroxyanilide;
4-methoxybenzoic acid-N-hydroxyanilide;
N,N'-dihydroxy-N,N'-diphenylterephthalamide;
decanoic acid-N-hydroxyanilide;
N-hydroxy-4-cyanoacetanilide;
N-hydroxy-4-acetylacetanilide;
N-hydroxy-4-hydroxyacetanilide;
N-hydroxy-3-(N'-hydroxyacetamide)acetanilide;
4-cyanobenzoic acid-N-hydroxyanilide;
N-hydroxy-4-nitroacetanilide;
N-hydroxyacetanilide;
N-hydroxy-N-phenyl-carbamic acid isopropyl ester;
N-hydroxy-N-phenyl-carbamic acid methyl ester;
N-hydroxy-N-phenyl-carbamic acid phenyl ester;
N-hydroxy-N-phenyl-carbamic acid ethyl ester; and
N-hydroxy-N-(4-cyanophenyl)-carbamic acid methyl ester.

7. The method according to any of claims 1-6, in which the laccase is a microbial laccase.

8. The method according to claim 7, wherein the laccase is derived from *Coprinus, Myceliophthora, Polyporus, Pycnoporus, Scytalidium* or *Rhizoctonia.*

9. The method according to claim 8, wherein the laccase is derived from *Coprinus cinereus, Myceliophthora thermophila, Polyporus pinsitus, Pycnoporus cinnabarinus, Scytalidium thermophilum* or *Rhizoctonia solani*.

10. The method according to any of claims 1-6, wherein the composition is an aqueous composition.

11. The method according to claim 10, wherein the concentration of the phenol oxidizing enzyme is in the range of from 0.001-10 mg enzyme protein per liter.

12. The method according to claim 10, wherein the concentration of the enhancing agent corresponds to 1-1000 µM.

13. The method according to any of claims 1-6, wherein said composition is a granulate.

14. A detergent composition comprising a surfactant and the composition described in any of claims 1-6.

15. A method of inhibiting micro-organisms present in laundry, wherein the laundry is treated with a soaking, washing or rinsing liquor comprising the composition described in any of claims 1-6.

16. A method of preserving a cosmetic product, wherein an effective amount of the composition described in any of claims 1-6 is incorporated into the cosmetic product.

17. The method according to claim 16, wherein the cosmetic product is a mouth wash composition, a cosmetic liquid or gel or paste, an eye lotion, an antiperspirant, a deodorant, a nasal spray, an eye ointment, an ointment or cream, a foot bath salt.

18. Use of the composition described in any of claims 1-6 for cleaning or disinfection of contact lenses.

19. A method of cleaning, disinfecting or inhibiting microbial growth on a hard surface, which is not oxygen-delignified softwood, wherein the surface is contacted with the composition described in any of claims 1-6.

20. The method according to claim 19, wherein the hard surface is a process equipment such as a member of a cooling tower, a water treatment plant, a dairy, a food processing plant, a chemical or pharmaceutical process plant.

21. The method according to claim 19, wherein the hard surface is a surface of water sanitation equipment.

22. The method according to claim 19, wherein the hard surface is a surface of equipment for paper pulp processing.

23. Use of the composition described in any of claims 1-6 in a cleaning-in-place system.

24. Use of the composition described in any of claims 1-6 for disinfection of water systems.

25. The use according to claim 24 for disinfection of water systems in paper pulp processing.

26. Use of the composition described in any of claims 1-6 for preserving paint.

27. An enzymatic antimicrobial composition comprising a laccase or a laccase related enzyme together with oxygen and an enhancing agent of the following formula: 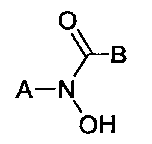 in which A and B independently of each other are: 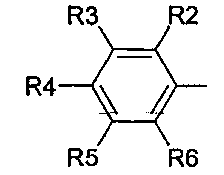 or B is H or C₁₋₁₆-alkyl, said alkyl may contain hydroxy, ester or ether groups, and R2, R3, R4, R5 and R6 independently of each other are H, OH, NH₂, COOH, SO₃H, C₁₋₁₂-alkyl, acyl, NO₂, CN, Cl, Br, F, CF₃, NOH-CO-phenyl, CO-NOH-phenyl, C₁₋₆-CO-NOH-A, CO-NOH-A, COR12, phenyl-CO-NOH-A, OR7, NR8R9, COOR10, or NOH-CO-R11, wherein R7, R8, R9, R10, R11 and R12 are C₁₋₁₂-alkyl or acyl; wherein the composition in an aqueous solution has a pH in the range of pH 5 to 10.5.

28. An enzymatic antimicrobial composition according to claim 27, in which A and B of the enhancing agent independently of each other are: 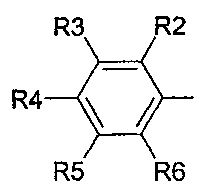 or B is H or C₁₋₃-alkyl, said alkyl may contain hydroxy, ester or ether groups, and R2, R3, R4, R5 and R6 independently of each other are H, OH, NH₂, COOH, SO₃H, C₁₋₃-alkyl, acyl, NO₂, CN, Cl, Br, F, CF₃, NOH-CO-phenyl, CO-NOH-phenyl, COR12, OR7, NR8R9, COOR10, or NOH-CO-R11, wherein R7, R8 and R9 are C₁₋₃-alkyl or acyl, and R10, R11 and R12 are C₁₋₃-alkyl.

29. An enzymatic antimicrobial composition according to claim 27, in which A and B of the enhancing agent independently of each other are: 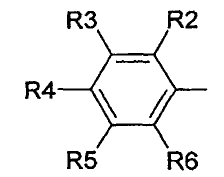 or B is H or C₁₋₃-alkyl, said alkyl may contain hydroxy or ether groups, and R2, R3, R4, R5 and R6 independently of each other are H, OH, NH₂, COOH, SO₃H, CH₃, acyl, NO₂, CN, Cl, Br, F, CF₃, CO-NOH-phenyl, COCH₃, OR7, NR8R9, or COOCH₃, wherein R7, R8 and R9 are CH₃ or COCH₃.

30. An enzymatic antimicrobial composition according to claim 27, in which A and B of the enhancing agent independently of each other are: 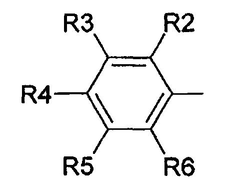 or B is H or C₁₋₃-alkyl, said alkyl may contain hydroxy or ether groups, and R2, R3, R4, R5 and R6 independently of each other are H, OH, COOH, SO₃H, CH₃, acyl, NO₂, CN, Cl, Br, F, CO-NOH-phenyl, OCH₃, COCH₃, or COOCH₃.

31. An enzymatic antimicrobial composition according to claim 27, in which A and B of the enhancing agent independently of each other are: 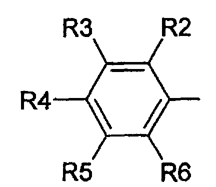 or B is C₁₋₃-alkyl, said alkyl may contain ether groups, and R2, R3, R4, R5 and R6 independently of each other are H, OH, COOH, SO₃H, CH₃, NO₂, CN, Cl, Br, CO-NOH-phenyl, or OCH₃.

32. A composition according to claim 27, in which the enhancing agent is selected from the group consisting of
4-nitrobenzoic acid-N-hydroxyanilide; 4-methoxybenzoic acid-N-hydroxyanilide; N,N'-dihydroxy-N,N'-diphenylterephthalamide; decanoic acid-N-hydroxyanilide; N-hydroxy-4-cyanoacetanilide; N-hydroxy-4-acetylacetanilide; N-hydroxy-4-hydroxyacetanilide; N-hydroxy-3-(N'-hydroxyacetamide)acetaniiide; 4-cyanobenzoic acid-N-hydroxyanilide; N-hydroxy-4-nitroacetanilide; N-hydroxyacetanilide; N-hydroxy-N-phenyl-carbamic acid isopropyl ester; N-hydroxy-N-phenyl-carbamic acid methyl ester; N-hydroxy-N-phenyl-carbamic acid phenyl ester; N-hydroxy-N-phenyl-carbamic acid ethyl ester; and N-hydroxy-N-(4-cyanophenyl)-carbamic acid methyl ester.

33. A composition according to any of claims 27-32, in which the laccase is a microbial laccase.

34. A composition according to claim 33, wherein the laccase is derived from *Coprinus, Myceliophthora, Polyporus, Pycnoporus, Scytalidium* or *Rhizoctonia.*

35. The composition according to any of claims 27-32, wherein said composition is an aqueous composition.

## Patentansprüche

1. Verfahren zum Abtöten oder Hemmen von Mikroorganismenzellen, bei dem man die Mikroorganismenzellen mit einer Zusammensetzung behandelt, die eine Laccase oder ein mit Laccase verwandtes Enzym gemeinsam mit Sauerstoff und einem Fördermittel der Formel: 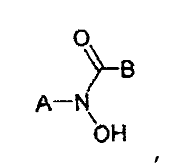 in der A und B unabhängig voneinander 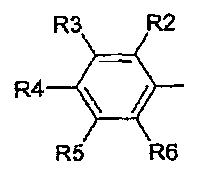 bedeuten
oder B H oder C₁₋₁₆-Alkyl bedeutet, wobei das Alkyl Hydroxy-, Ester- oder Ethergruppen enthalten kann, und R2, R3, R4, R5 und R6 unabhängig voneinander H, OH, NH₂, COOH, SO₃H, C₁₋₁₂-Alkyl, Acyl, NO₂, CN, Cl, Br, F, CF₃, NOH-CO-Phenyl, CO-NOH-Phenyl, C₁₋₆-CO-NOH-A, CO-NOH-A, COR12, Phenyl-CO-NOH-A, OR7, NR8R9, COOR10 oder NOH-CO-R11 bedeuten, wobei R7, R8, R9, R10, R11 und R12 C₁₋₁₂-Alkyl oder Acyl bedeuten,
enthält.

2. Verfahren nach Anspruch 1, bei dem A und B des Fördermittels unabhängig voneinander 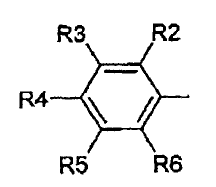 bedeuten
oder B H oder C₁₋₃-Alkyl bedeutet, wobei das Alkyl Hydroxy-, Ester- oder Ethergruppen enthalten kann, und R2, R3, R4, R5 und R6 unabhängig voneinander H, OH, NH₂, COOH, SO₃H, C₁₋₃-Alkyl, Acyl, NO₂, CN, Cl, Br, F, CF₃, NOH-CO-Phenyl, CO-NOH-Phenyl, COR12, OR7, NR8R9, COOR10 oder NOH-CO-R11 bedeuten, wobei R7, R8 und R9 C₁₋₃-Alkyl oder Acyl bedeuten und R10, R11 und R12 C₁₋₃-Alkyl bedeuten.

3. Verfahren nach Anspruch 1, bei dem A und B des Fördermittels unabhängig voneinander 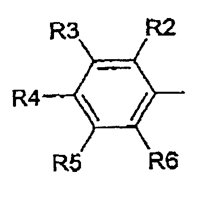 bedeuten
oder B H oder C₁₋₃-Alkyl bedeutet, wobei das Alkyl Hydroxy- oder Ethergruppen enthalten kann, und R2, R3, R4, R5 und R6 unabhängig voneinander H, OH, NH₂, COOH, SO₃H, CH₃, Acyl, NO₂, CN, Cl, Br, F, CF₃, CO-NOH-Phenyl, COCH₃, OR7, NR8R9 oder COOCH₃ bedeuten, wobei R7, R8 und R9 CH₃ oder COCH₃ bedeuten.

4. Verfahren nach Anspruch 1, bei dem A und B des Fördermittels unabhängig voneinander 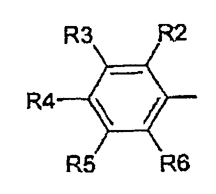 bedeuten
oder B H oder C₁₋₃-Alkyl bedeutet, wobei das Alkyl Hydroxy- oder Ethergruppen enthalten kann, und R2, R3, R4, R5 und R6 unabhängig voneinander H, OH, COOH, SO₃H, CH₃, Acyl, NO₂, CN, Cl, Br, F, CO-NOH-Phenyl, OCH₃, COCH₃ oder COOCH₃ bedeuten.

5. Verfahren nach Anspruch 1, bei dem A und B des Fördermittels unabhängig voneinander 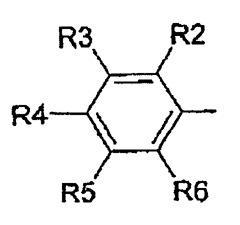 bedeuten
oder B C₁₋₃-Alkyl bedeutet, wobei das Alkyl Ethergruppen enthalten kann, und R2, R3, R4, R5 und R6 unabhängig voneinander H, OH, COOH, SO₃H, CH₃, NO₂, CN, Cl, Br, CO-NOH-Phenyl oder OCH₃ bedeuten.

6. Verfahren nach Anspruch 1, bei dem das Fördermittel aus der Gruppe
4-Nitrobenzoesäure-N-hydroxyanilid,
4-Methoxybenzoesäure-N-hydroxyanilid,
N,N'-Dihydroxy-N,N'-diphenylterephthalamid,
Decansäure-N-hydroxyanilid,
N-Hydroxy-4-cyanacetanilid,
N-Hydroxy-4-acetylacetanilid,
N-Hydroxy-4-hydroxyacetanilid,
N-Hydroxy-3-(N'-hydroxyacetamid)acetanilid,
4-Cyanbenzoesäure-N-hydroxyanilid,
N-Hydroxy-4-nitroacetanilid,
N-Hydroxyacetanilid,
N-Hydroxy-N-phenylcarbaminsäureisopropylester,
N-Hydroxy-N-phenylcarbaminsäuremethylester,
N-Hydroxy-N-phenylcarbaminsäurephenylester,
N-Hydroxy-N-phenylcarbaminsäureethylester und
N-Hydroxy-N-(4-cyanphenyl)carbaminsäuremethylester
stammt.

7. Verfahren nach einem der Ansprüche 1-6, wobei es sich bei der Laccase um eine mikrobielle Laccase handelt.

8. Verfahren nach Anspruch 7, wobei die Laccase aus *Coprinus, Myceliophthora, Polyporus, Pycnoporus, Scytalidium* oder *Rhizoctonia* gewonnen wird.

9. Verfahren nach Anspruch 8, wobei die Laccase aus *Coprinus cinereus, Myceliophthora thermophila, Polyporus pinsitus, Pycnoporus cinnabarinus, Scytalidium thermophilum* oder *Rhizoctonia solani* gewonnen wird.

10. Verfahren nach einem der Ansprüche 1-6, wobei es sich bei der Zusammensetzung um eine wäßrige Zusammensetzung handelt.

11. Verfahren nach Anspruch 10, wobei die Konzentration des phenoloxidierenden Enzyms im Bereich von 0,001-10 mg Enzymprotein pro Liter liegt.

12. Verfahren nach Anspruch 10, wobei die Konzentration des Fördermittels 1-1000 µm entspricht.

13. Verfahren nach einem der Ansprüche 1-6, wobei es sich bei der Zusammensetzung um ein Granulat handelt.

14. Reinigungszusammensetzung mit einem Tensid und der in einem der Ansprüche 1-6 beschriebenen Zusammensetzung.

15. Verfahren zur Hemmung von in Wäsche vorhandenen Mikroorganismen, wobei die Wäsche mit einer Einweich-, Wasch- oder Spülflüssigkeit mit der Zusammensetzung nach einem der Ansprüche 1-6 behandelt wird.

16. Verfahren zur Konservierung eines Kosmetikprodukts, wobei eine wirksame Menge der in einem der Ansprüche 1-6 beschriebenen Zusammensetzung in das Kosmetikprodukt eingearbeitet wird.

17. Verfahren nach Anspruch 16, wobei es sich bei dem Kosmetikprodukt um ein Mundwasser, eine Kosmetikflüssigkeit, ein Kosmetikgel, eine Kosmetikpaste, eine Augenlotion, ein Antiperspirant, ein Deodorant, ein Nasenspray, eine Augensalbe, eine Salbe oder Creme oder ein Fußbadesalz handelt.

18. Verwendung der Zusammensetzung nach einem der Ansprüche 1-6 zur Reinigung oder Desinfektion von Kontaktlinsen.

19. Verfahren zum Reinigen, Desinfizieren oder Hemmen von Mikroorganismenwachstum auf einer harten Oberfläche, bei der es sich nicht um mit Sauerstoff delignifiziertes Weichholz handelt, wobei die Oberfläche mit der Zusammensetzung nach einem der Ansprüche 1-6 in Kontakt gebracht wird.

20. Verfahren nach Anspruch 19, wobei es sich bei der harten Oberfläche um ein in einem Prozeß verwendetes Gerät, wie einen Bestandteil eines Kühlturms, eine Kläranlage, eine Molkerei, eine in der Nahrungsmittelverarbeitung verwendete Anlage oder eine Anlage für ein chemisches oder pharmazeutisches Verfahren handelt.

21. Verfahren nach Anspruch 19, wobei es sich bei der harten Oberfläche um eine Oberfläche von Wasserhygienisierungsgeräten handelt.

22. Verfahren nach Anspruch 19, wobei es sich bei der harten Oberfläche um eine Oberfläche von in der Papierzellstoffverarbeitung verwendeten Geräten handelt.

23. Verwendung der Zusammensetzung nach einem der Ansprüche 1-6 in einem Vor-Ort-Reinigungssystem.

24. Verwendung der Zusammensetzung nach einem der Ansprüche 1-6 zur Desinfektion von Wassersystemen.

25. Verwendung nach Anspruch 24 zur Desinfektion von Wassersystemen in der Papierzellstoffverarbeitung.

26. Verwendung der Zusammensetzung nach einem der Ansprüche 1-6 zur Konservierung von Anstrichmitteln.

27. Antimikrobielle Enzymzusammensetzung mit einer Laccase oder einem mit Laccase verwandten Enzym gemeinsam mit Sauerstoff und einem Fördermittel der Formel: 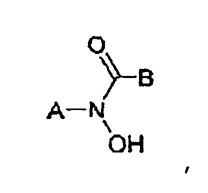 in der A und B unabhängig voneinander 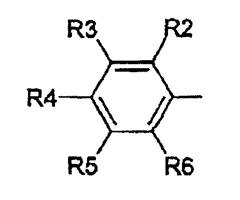 bedeuten
oder B H oder C₁₋₁₆-Alkyl bedeutet, wobei das Alkyl Hydroxy-, Ester- oder Ethergruppen enthalten kann, und R2, R3, R4, R5 und R6 unabhängig voneinander H, OH, NH₂, COOH, SO₃H, C₁₋₁₂-Alkyl, Acyl, NO₂, CN, Cl, Br, F, CF₃, NOH-CO-Phenyl, CO-NOH-Phenyl, C₁₋₆-CO-NOH-A, CO-NOH-A, COR12, Phenyl-CO-NOH-A, OR7, NR8R9, COOR10 oder NOH-CO-R11 bedeuten, wobei R7, R8, R9, R10, R11 und R12 C₁₋₁₂-Alkyl oder Acyl bedeuten,
enthält, wobei die Zusammensetzung in wäßriger Lösung einen pH-Wert im Bereich von pH 5 bis 10,5 aufweist.

28. Antimikrobielle Enzymzusammensetzung nach Anspruch 27, in der A und B des Fördermittels unabhängig voneinander 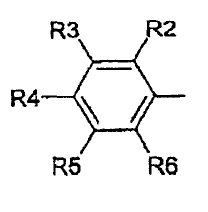 bedeuten
oder B H oder C₁₋₃-Alkyl bedeutet, wobei das Alkyl Hydroxy-, Ester- oder Ethergruppen enthalten kann, und R2, R3, R4, R5 und R6 unabhängig voneinander H, OH, NH₂, COOH, SO₃H, C₁₋₃-Alkyl, Acyl, NO₂, CN, Cl, Br, F, CF₃, NOH-CO-Phenyl, CO-NOH-Phenyl, COR12, OR7, NR8R9, COOR10 oder NOH-CO-R11 bedeuten, wobei R7, R8 und R9 C₁₋₃-Alkyl oder Acyl bedeuten und R10, R11 und R12 C₁₋₃-Alkyl bedeuten.

29. Antimikrobielle Enzymzusammensetzung nach Anspruch 27, in der A und B des Fördermittels unabhängig voneinander 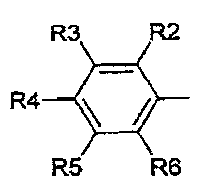 bedeuten
oder B H oder C₁₋₃-Alkyl bedeutet, wobei das Alkyl Hydroxy- oder Ethergruppen enthalten kann, und R2, R3, R4, R5 und R6 unabhängig voneinander H, OH, NH₂, COOH, SO₃H, CH₃, Acyl, NO₂, CN, Cl, Br, F, CF₃, CO-NOH-Phenyl, COCH₃, OR7, NR8R9 oder COOCH₃ bedeuten, wobei R7, R8 und R9 CH₃ oder COCH₃ bedeuten.

30. Antimikrobielle Enzymzusammensetzung nach Anspruch 27, in der A und B des Fördermittels unabhängig voneinander 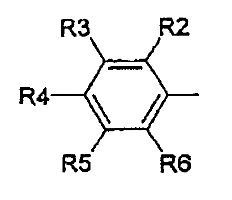 bedeuten
oder B H oder C₁₋₃-Alkyl bedeutet, wobei das Alkyl Hydroxy- oder Ethergruppen enthalten kann, und R2, R3, R4, R5 und R6 unabhängig voneinander H, OH, COOH, SO₃H, CH₃, Acyl, NO₂, CN, Cl, Br, F, CO-NOH-Phenyl, OCH₃, COCH₃ oder COOCH₃ bedeuten.

31. Antimikrobielle Enzymzusammensetzung nach Anspruch 27, in der A und B des Fördermittels unabhängig voneinander 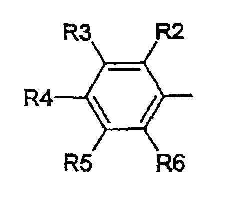 bedeuten
oder B C₁₋₃-Alkyl bedeutet, wobei das Alkyl Ethergruppen enthalten kann, und R2, R3, R4, R5 und R6 unabhängig voneinander H, OH, COOH, SO₃H, CH₃, NO₂, CN, Cl, Br, CO-NOH-Phenyl oder OCH₃ bedeuten.

32. Zusammensetzung nach Anspruch 27, in der das Fördermittel aus der Gruppe
4-Nitrobenzoesäure-N-hydroxyanilid,
4-Methoxybenzoesäure-N-hydroxyanilid,
N,N'-Dihydroxy-N,N'-diphenylterephthalamid,
Decansäure-N-hydroxyanilid,
N-Hydroxy-4-cyanacetanilid,
N-Hydroxy-4-acetylacetanilid,
N-Hydroxy-4-hydroxyacetanilid,
N-Hydroxy-3-(N'-hydroxyacetamid)acetanilid,
4-Cyanbenzoesäure-N-hydroxyanilid,
N-Hydroxy-4-nitroacetanilid,
N-Hydroxyacetanilid,
N-Hydroxy-N-phenylcarbaminsäureisopropylester,
N-Hydroxy-N-phenylcarbaminsäuremethylester,
N-Hydroxy-N-phenylcarbaminsäurephenylester,
N-Hydroxy-N-phenylcarbaminsäureethylester und
N-Hydroxy-N-(4-cyanphenyl)carbaminsäuremethylester
stammt.

33. Zusammensetzung nach einem der Ansprüche 27-32, wobei es sich bei der Laccase um eine mikrobielle Laccase handelt.

34. Zusammensetzung nach Anspruch 33, wobei die Laccase aus *Coprinus, Myceliophthora, Polyporus, Pycnoporus, Scytalidium* oder *Rhizoctonia* gewonnen wird.

35. Zusammensetzung nach einem der Ansprüche 27-32, wobei es sich bei der Zusammensetzung um eine wäßrige Zusammensetzung handelt.

## Revendications

1. Procédé pour tuer ou inhiber des cellules microbiennes, comprenant le traitement desdites cellules microbiennes avec une composition comprenant une laccase ou une enzyme apparentée à une laccase conjointement avec de l'oxygène et un agent amplificateur ayant la formule suivante : 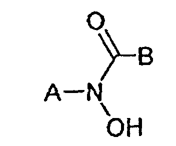 dans laquelle A et B sont indépendamment l'un de l'autre : 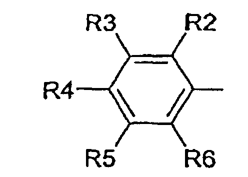 ou B est H ou un groupe alkyle en C₁₋₁₆, ledit groupe alkyle pouvant contenir des groupes hydroxy, ester ou éther, et R2, R3, R4, R5 et R6 sont, indépendamment les uns des autres, H, OH, NH₂, COOH, SO₃H, alkyle en C₁₋₁₂, acyle, NO₂, CN, Cl, Br, F, CF₃, NOH-CO-phényle, CO-NOH-phényle, C₁₋₆-CO-NOH-A, CO-NOH-A, COR12, phényl-CO-NOH-A, OR7, NR8R9, COOR10 ou NOH-CO-R11, où R7, R8, R9, R10, R11 et R12 sont un groupe alkyle en C₁₋₁₂ ou acyle.

2. Procédé selon la revendication 1, dans lequel A et B de l'agent amplificateur sont indépendamment l'un de l'autre : 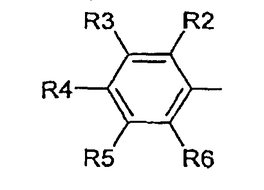 ou B est H ou un groupe alkyle en C₁₋₃, ledit groupe alkyle pouvant contenir des groupes hydroxy, ester ou éther, et R2, R3, R4, R5 et R6 sont, indépendamment les uns des autres, H, OH, NH₂, COOH, SO₃H, alkyle en C₁₋₃, acyle, NO₂, CN, Cl, Br, F, CF₃, NOH-CO-phényle, CO-NOH-phényle, COR12, OR7, NR8R9, COOR10 ou NOH-CO-R11, où R7, R8 et R9 sont un groupe alkyle en C₁₋₃ ou acyle, et R10, R11 et R12 sont un groupe alkyle en C₁₋₃.

3. Procédé selon la revendication 1, dans lequel A et B de l'agent amplificateur sont indépendamment l'un de l'autre : 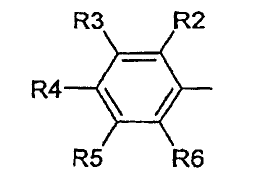 ou B est H ou un groupe alkyle en C₁₋₃, ledit groupe alkyle pouvant contenir des groupes hydroxy ou éther, et R2, R3, R4, R5 et R6 sont, indépendamment les uns des autres, H, OH, NH₂, COOH, SO₃H, CH₃, acyle, NO₂, CN, Cl, Br, F, CF₃, CO-NOH-phényle, COCH₃, OR7, NR8R9 ou COOCH₃, où R7, R8 et R9 sont CH₃ ou COCH₃.

4. Procédé selon la revendication 1, dans lequel A et B de l'agent amplificateur sont indépendamment l'un de l'autre : 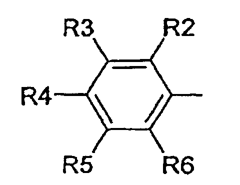 ou B est H ou un groupe alkyle en C₁₋₃, ledit groupe alkyle pouvant contenir des groupes hydroxy ou éther, et R2, R3, R4, R5 et R6 sont, indépendamment les uns des autres, H, OH, COOH, SO₃H, CH₃, acyle, NO₂, CN, Cl, Br, F, CO-NOH-phényle, OCH₃, COCH₃ ou COOCH₃.

5. Procédé selon la revendication 1, dans lequel A et B de l'agent amplificateur sont indépendamment l'un de l'autre : 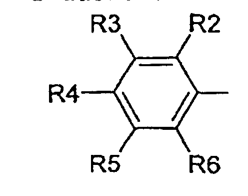 ou B est un groupe alkyle en C₁₋₃, ledit groupe alkyle pouvant contenir des groupes éther, et R2, R3, R4, R5 et R6 sont, indépendamment les uns des autres, H, OH, COOH, SO₃H, CH₃, NO₂, CN, Cl, Br, CO-NOH-phényle ou OCH₃.

6. Procédé selon la revendication 1, dans lequel l'agent amplificateur est choisi dans l'ensemble constitué de
acide 4-nitrobenzoïque-N-hydroxyanilide ;
acide 4-méthoxybenzoïque-N-hydroxyanilide;
N,N'-dihydroxy-N,N'-diphényltéréphtalamide ;
acide décanoïque-N-hydroxyanilide ;
N-hydroxy-4-cyanoacétanilide ;
N-hydroxy-4-acétylacétanilide ;
N-hydroxy-4-hydroxyacétanilide ;
N-hydroxy-3-(N'-hydroxyacétamide)acétanilide ;
acide 4-cyanobenzoique-N-hydroxyanilide ;
N-hydroxy-4-nitroacétanilide ;
N-hydroxyacétanilide ;
ester isopropylique de l'acide N-hydroxy-N-phényl-carbamique ;
ester méthylique de l'acide N-hydroxy-N-phényl-carbamique ;
ester phénylique de l'acide N-hydroxy-N-phényl-carbamique ;
ester éthylique de l'acide N-hydroxy-N-phényl-carbamique ; et
ester méthylique de l'acide N-hydroxy-N-(4-cyanophényl)-carbamique.

7. Procédé selon l'une quelconque des revendications 1 à 6, dans lequel la laccase est une laccase microbienne.

8. Procédé selon la revendication 7, dans lequel la laccase provient de *Coprinus, Myceliophtora, Polyporus, Pycnoporus, Scytalidium* ou *Rhizoctonia.*

9. Procédé selon la revendication 8, dans lequel la laccase provient de *Coprinus cinereus, Myceliophtora thermophila, Polyporus pinsitus, Pycnoporus cinnabarinus, Scytalidium thermophilum* ou *Rhizoctonia solani.*

10. Procédé selon l'une quelconque des revendications 1 à 6, dans lequel la composition est une composition aqueuse.

11. Procédé selon la revendication 10, dans lequel la concentration de l'enzyme oxydant le phénol est dans la plage de 0,001 à 10 mg de protéine enzymatique par litre.

12. Procédé selon la revendication 10, dans lequel la concentration de l'agent amplificateur correspond à 1-1000 µM.

13. Procédé selon l'une quelconque des revendications 1 à 6, dans lequel ladite composition est un granulat.

14. Composition détergente comprenant un agent tensioactif et la composition décrite dans l'une quelconque des revendications 1 à 6.

15. Procédé d'inhibition de micro-organismes présents dans une blanchisserie, dans lequel on traite la blanchisserie avec une lessive de trempage, de lavage ou de rinçage comprenant la composition décrite dans l'une quelconque des revendications 1 à 6.

16. Procédé de conservation d'un produit cosmétique, dans lequel on incorpore une quantité efficace de la composition décrite dans l'une quelconque des revendications 1 à 6 dans le produit cosmétique.

17. Procédé selon la revendication 16, dans lequel le produit cosmétique est une composition pour bains de bouche, un liquide ou un gel ou une pâte cosmétique, une lotion pour les yeux, un anti-transpirant, un déodorant, une pulvérisation nasale, un onguent pour les yeux, un onguent ou une crème, un sel pour bains de pieds.

18. Utilisation de la composition décrite dans l'une quelconque des revendications 1 à 6 pour nettoyer ou désinfecter des lentilles de contact.

19. Procédé de nettoyage, de désinfection ou d'inhibition de la prolifération microbienne sur une surface dure qui n'est pas du bois tendre délignifié avec de l'oxygène, dans lequel on amène la surface au contact de la composition décrite dans l'une quelconque des revendications 1 à 6.

20. Procédé selon la revendication 19, dans lequel la surface dure est un équipement industriel comme un élément d'une tour de refroidissement, une usine de traitement des eaux, une laiterie, une usine de transformation des aliments, une installation industrielle chimique ou pharmaceutique.

21. Procédé selon la revendication 19, dans lequel la surface dure est une surface d'un équipement d'assainissement des eaux.

22. Procédé selon la revendication 19, dans lequel la surface dure est une surface d'un équipement pour le traitement de la pâte à papier.

23. Utilisation de la composition décrite dans l'une quelconque des revendications 1 à 6 dans un système de nettoyage en place.

24. Utilisation de la composition décrite dans l'une quelconque des revendications 1 à 6 pour la désinfection de systèmes d'alimentation en eau.

25. Utilisation selon la revendication 24 pour la désinfection de systèmes d'alimentation en eau dans le traitement de la pâte à papier.

26. Utilisation de la composition décrite dans l'une quelconque des revendications 1 à 6 pour conserver la peinture.

27. Composition enzymatique antimicrobienne comprenant une laccase ou une enzyme apparentée à une laccase conjointement avec de l'oxygène et un agent amplificateur ayant la formule suivante : 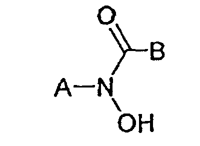 dans laquelle A et B sont indépendamment l'un de l'autre : 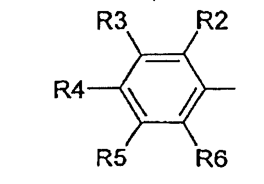 ou B est H ou un groupe alkyle en C₁₋₁₆, ledit groupe alkyle pouvant contenir des groupes hydroxy, ester ou éther, et R2, R3, R4, R5 et R6 sont, indépendamment les uns des autres, H, OH, NH₂, COOH, SO₃H, alkyle en C₁₋₁₂, acyle, NO₂, CN, Cl, Br, F, CF₃, NOH-CO-phényle, CO-NOH-phényle, C₁₋₆-CO-NOH-A, CO-NOH-A, COR12, phényl-CO-NOH-A, OR7, NR8R9, COOR10 ou NOH-CO-R11, où R7, R8, R9, R10, R11 et R12 sont un groupe alkyle en C₁₋₁₂ ou acyle ;ladite composition en solution aqueuse ayant un pH dans la plage de pH 5 à 10,5.

28. Composition enzymatique antimicrobienne selon la revendication 27, dans laquelle A et B de l'agent amplificateur sont indépendamment l'un de l'autre : 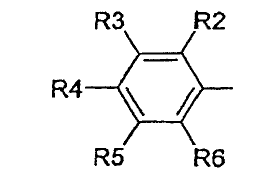 ou B est H ou un groupe alkyle en C₁₋₃, ledit groupe alkyle pouvant contenir des groupes hydroxy, ester ou éther, et R2, R3, R4, R5 et R6 sont, indépendamment les uns des autres, H, OH, NH₂, COOH, SO₃H, alkyle en C₁₋₃, acyle, NO₂, CN, Cl, Br, F, CF₃, NOH-CO-phényle, CO-NOH-phényle, COR12, OR7, NR8R9, COOR10 ou NOH-CO-R11, où R7, R8 et R9 sont un groupe alkyle en C₁₋₃ ou acyle, et R10, R11 et R12 sont un groupe alkyle en C₁₋₃.

29. Composition enzymatique antimicrobienne selon la revendication 27, dans laquelle A et B de l'agent amplificateur sont indépendamment l'un de l'autre : 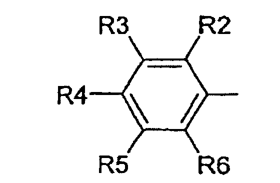 ou B est H ou un groupe alkyle en C₁₋₃, ledit groupe alkyle pouvant contenir des groupes hydroxy ou éther, et R2, R3, R4, R5 et R6 sont, indépendamment les uns des autres, H, OH, NH₂, COOH, SO₃H, CH₃, acyle, NO₂, CN, Cl, Br, F, CF₃, CO-NOH-phényle, COCH₃, OR7, NR8R9 ou COOCH₃, où R7, R8 et R9 sont CH₃ ou COCH₃.

30. Composition enzymatique antimicrobienne selon la revendication 27, dans laquelle A et B de l'agent amplificateur sont indépendamment l'un de l'autre : 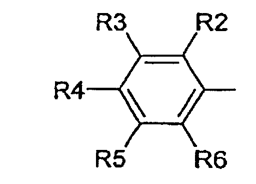 ou B est H ou un groupe alkyle en C₁₋₃, ledit groupe alkyle pouvant contenir des groupes hydroxy ou éther, et R2, R3, R4, R5 et R6 sont, indépendamment les uns des autres, H, OH, COOH, SO₃H, CH₃, acyle, NO₂, CN, Cl, Br, F, CO-NOH-phényle, OCH₃, COCH₃ ou COOCH₃.

31. Composition enzymatique antimicrobienne selon la revendication 27, dans laquelle A et B de l'agent amplificateur sont indépendamment l'un de l'autre : 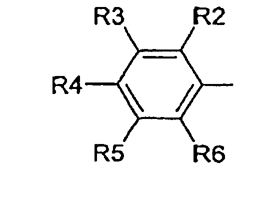 ou B est un groupe alkyle en C₁₋₃, ledit groupe alkyle pouvant contenir des groupes d'éther, et R2, R3, R4, R5 et R6 sont, indépendamment les uns des autres, H, OH, COOH, SO₃H, CH₃, NO₂, CN, Cl, Br, CO-NOH-phényle ou OCH₃.

32. Composition selon la revendication 27, dans laquelle l'agent amplificateur est choisi dans l'ensemble constitué de
acide 4-nitrobenzoïque-N-hydroxyanilide ;
acide 4-méthoxybenzoïque-N-hydroxyanilide ;
N,N'-dihydroxy-N,N'-diphényltéréphtalamide ;
acide décanoïque-N-hydroxyanilide ;
N-hydroxy-4-cyanoacétanilide ;
N-hydroxy-4-acétylacétanilide ;
N-hydroxy-4-hydroxyacétanilide ;
N-hydroxy-3-(N'-hydroxyacétamide)acétanilide ;
acide 4-cyanobenzoïque-N-hydroxyanilide ;
N-hydroxy-4-nitroacétanilide ;
N-hydroxyacétanilide ;
ester isopropylique de l'acide N-hydroxy-N-phényl-carbamique ;
ester méthylique de l'acide N-hydroxy-N-phényl-carbamique ;
ester phénylique de l'acide N-hydroxy-N-phényl-carbamique ;
ester éthylique de l'acide N-hydroxy-N-phényl-carbamique ; et
ester méthylique de l'acide N-hydroxy-N-(4-cyanophényl)-carbamique.

33. Composition selon l'une quelconque des revendications 27 à 32, dans laquelle la laccase est une laccase microbienne.

34. Composition selon la revendication 33, dans laquelle la laccase provient de *Coprinus, Myceliophtora, Polyporus, Pycnoporus, Scytalidium* ou *Rhizoctonia.*

35. Composition selon l'une quelconque des revendications 27 à 32, ladite composition étant une composition aqueuse.
